# EUROPEAN PATENT SPECIFICATION

(11) **EP 2 416 662 B1**
(45) Date of publication and mention of the grant of the patent: **25.03.2015**
(21) Application number: 10762452.0
(22) Date of filing: 08.04.2010
(51) Int. Cl.: A01N 63/00, A61K 39/12

(54) **ALPHAVIRUS REPLICON PARTICLES EXPRESSING TRP2**
TRP2 EXPRIMIERENDE ALPHAVIRUS-REPLIKON-PARTIKEL
PARTICULES DE RÉPLICON D'ALPHAVIRUS EXPRIMANT TRP2

(30) Priority: 08.04.2009 US 167774 P
(43) Date of publication of application: 15.02.2012
(73) Proprietor: Alphavax, Inc., Research Triangle Park, NC 27709 (US); Sloan-Kettering Institute for Cancer Research, New York, NY 10065 (US)
(72) Inventor: WOLCHOK, Jedd D., New York New York 10016 (US); AVOGADRI, Francesca, New York New York 10044 (US); OLMSTED, Robert A., Chapel Hill North Carolina 27517 (US); MAUGHAN, Maureen, Chapel Hill North Carolina 27514 (US)
(74) Representative: O'Connell, Maura
(86) International application number: PCT/US2010/030423
(87) International publication number: WO 2010/118252

(56) References cited:
- US-A1- 2005 266 550
- US-A1- 2006 099 587
- GOLDBERG S M ET AL: "Comparison of two cancer vaccines targeting tyrosinase: Plasmid DNA and recombinant alphavirus replicon particles", CLINICAL CANCER RESEARCH 20051115 US, vol. 11, no. 22, 15 November 2005 (2005-11-15), pages 8114-8121, XP002689632, ISSN: 1078-0432
- GOLDBERG STACIE M ET AL: "A successful prime-boost immunization strategy in a tumor model: Targeting tyrosinase in melanoma.", PROCEEDINGS OF THE AMERICAN ASSOCIATION FOR CANCER RESEARCH ANNUAL MEETING, vol. 45, March 2004 (2004-03), page 288, XP002689633, & 95TH ANNUAL MEETING OF THE AMERICAN-ASSOCIATION-FOR-CANCER-RESEARCH; ORLANDO, FL, USA; MARCH 27 -31, 2004 ISSN: 0197-016X
- YAMANAKA RYUYA ET AL: "Induction of antigen-specific immune responses against malignant brain tumors by intramuscular injection of sindbis DNA encoding gp100 and IL-18.", DNA AND CELL BIOLOGY MAY 2005, vol. 24, no. 5, May 2005 (2005-05), pages 317-324, XP002689634, ISSN: 1044-5498
- LEITNER WOLFGANG W ET AL: "Alphavirus-based DNA vaccine breaks immunological tolerance by activating innate antiviral pathways.", NATURE MEDICINE, vol. 9, no. 1, January 2003 (2003-01), pages 33-39, XP002689635, ISSN: 1078-8956
- AVOGADRI FRANCESCA ET AL: "Alphavirus replicon particles expressing TRP-2 provide potent therapeutic effect on melanoma through activation of humoral and cellular immunity.", PLOS ONE 2010, vol. 5, no. 9, 2010, XP002689636, ISSN: 1932-6203

## Description

### BACKGROUND OF THE INVENTION

The present invention relates to recombinant DNA technology, pharmaceutical compositions for prophylaxis or treatment of cancer, in particular, comprising alphavirus replicon particles containing an expressible coding sequence for a melanoma antigen of interest, and to methods for triggering or enhancing an immune response to a melanoma antigen, especially dopachrome tautomerase (DCT), which is also referred as tyrosinase-related protein (TRP2), introducing foreign nucleic acid(s) encoding a cancer antigen into a eukaryotic cell via alphavirus replicon particles, and more particularly, to the use of immunogenic compositions comprising infectious, propagation-defective virus particles or virus-like particles, especially Venezuelan Encephalitis Virus particles expressing TRP2, vaccines and melanoma therapy applications. In particular, the present disclosure provides alphavirus replicon particle (ARP) preparations, especially VEE particles expressing a melanoma antigen (in particular TRP2), for use in human and veterinary medicine and for enhancing or inducing the immune system's response to this antigen so that the incidence, metastasis and/or severity of melanoma is reduced.

Melanoma is an especially devastating disease, for which there is no approved vaccine for use in therapy or for prevention of melanoma. There has been relatively limited success in melanoma vaccine regimens or using immunogenic compositions in the treatment of this cancer if it has advanced.

The Alphavirus genus includes a variety of viruses, all of which are members of the Togaviridae family. The alphaviruses include Eastern Equine Encephalitis Virus (EEE), Venezuelan Equine Encephalitis Virus (VEE), Everglades Virus, Mucambo Virus, Pixuna Virus, Western Equine Encephalitis Virus (WEE), Sindbis Virus, Semliki Forest Virus, among others. The viral genome is a single-stranded, messenger-sense RNA, modified at the 5'-end with a methylated cap and at the 3'-end with a variable-length poly (A) tract. Structural subunits containing a single viral protein, capsid, associate with the RNA genome in an icosahedral nucleocapsid. In the virion, the capsid is surrounded by a lipid envelope covered with a regular array of transmembrane protein spikes, each of which consists of a heterodimeric complex of two glycoproteins, E1 and E2. See Pedersen et al., J. Virol 14:40 (1974). The Sindbis and Semliki Forest viruses are considered the prototypical alphaviruses and have been studied extensively. See Schlesinger, The Togaviridae and Flaviviridae, Plenum Publishing Corp., New York (1986). The VEE virus has been studied extensively, see, e.g., U.S. Patent No. 5,185,440.

The studies of these viruses have led to the development of techniques for vaccinating against the alphavirus diseases and against other diseases through the use of alphavirus vectors for the introduction of foreign genes. One such system is the alphavirus replicon system, as described in U.S. Patent No. 6,190,666 to Garoff et al., U.S. Patent Nos. 5,792,462, 6,521,235 and 6,156,558 to Johnston et al., U.S. Patent Nos. 5,814,482, 5,843,723, 5,789,245, 6,015,694, 6,105,686 and 6,376,236 to Dubensky et al; U.S. Patent Application No. 6,767,699 (Polo et al.), U.S. Patent Nos. 7,045,335, 7,425,337, and 7,442,381 (Smith et al. and U.S. Published Application 2009-0075384 (Kamrud et al.). Improved constructs, both helper(s) and replicon, for use in producing alphavirus replicon particles are described in U.S. Patent No. 7,045, 335 (Smith et al.) and WO 2004/085660 (Smith et al.), and novel processes for their manufacture are described in U.S. Patent No. 7,078,218 (Smith et al.). Additional vectors and strategies are described in U.S Published Application No. WO 2008/085557 and WO 2009/047255.

US Patent publication No. 2006/0099587 provides immunogenic compositions and methods that may be used to administer safer (i.e., attenuated) alphavirus vectors (such as alphavirus vectors comprising a VEE virion shell) that retain improved immunogenicity as compared with other attenuated alphaviruses. Goldberg S M et al (Clinical Cancer Research 20051115 US, vol. 11, no. 22, 15 November 2005, pages 8114-8121) compares two cancer vaccines targeting tyrosinase. Goldberg Stacie M et al (Proceedings Of The American Association For Cancer Research Annual Meeting, vol. 45, March 2004, page 288, & 95th Annual Meeting Of The American-Association-For-Cancer-Research; Orlando, FL, USA; March 27 -31, 2004) discloses immunization of certain tumor mice with human tyrosinase-related proteins 1 and 2. Yamanaka Ryuya et al (DNA And Cell Biology May 2005, vol. 24, no. 5, pages 317-324) discloses the construction of a Sindbis DNA expression vector and evaluates its potential for brain tumor therapy. Leitner Wolfgang W et al (Nature Medicine, vol. 9, no. 1, January 2003, pages 33-39) discloses an alphavirus-based DNA vaccine that activates innate antiviral pathways.

There remains a need in the art for methods and compositions for the treatment and prophylaxis of melanoma, an especially aggressive and deadly cancer affecting large numbers of people every year.

### BRIEF SUMMARY OF THE INVENTION

The present disclosure provides a pharmaceutical composition for use in enhancing or inducing an immune response to melanoma cells in the subject; an immunogenic composition; and a pharmaceutical composition for use in a method of reducing the risk of contracting melanoma in a human or animal subject or reducing the severity or delaying progression of melanoma in a human or animal subject with melanoma, as recited in the accompanying claims. The present disclosure discloses a method of enhancing an immune response to a melanoma antigen, in particular dopachrome tautomerase (DCT, TRP2) in a human or animal subject via the administration of a pharmaceutical composition comprising alphavirus replicon particles expressing TRP2 or an effective fragment thereof. In the present compositions for use and compositions, the TRP2 is desirably, but not necessarily, identical in species source to that of the subject to whom the composition is administered. In specific embodiments, the mouse TRP2 has the amino acid sequence as set for in Table 1 b and an exemplary human TRP2 has the amino acid sequence as set forth herein below. Additional coding sequences are given in herein below, and further sequences are known to the art.

Also provided herein are compositions for use in methods of preventing, reducing the likelihood of developing, reducing the severity of melanoma, reducing the time to progression after an initial intervention or increasing survival time after diagnosis of melanoma in a human or animal subject comprising administering a pharmaceutical comprising an effective amount of alphavirus replicon particles expressing the melanoma antigen dopachrome tautomerase (DCT, TRP2). The composition can be administered to the subject in any manner consistent with administration of immunogenic compositions, intraperitoneal, intramuscular, intradermal, intranasal, intravaginal, intrarectal, subcutaneous or intravenous, especially via the subcutaneous or intramuscular routes of administration. In certain situations, especially for treatment of prevention of metastatic melanoma, intravenous administration could be used. In an animal subject, footpad injection is another route of administration for the present compositions. In these methods, the administration is desirably repeated, although subsequent dosages of the TRP2 may be via DNA vaccine or recombinant protein as well as, or in addition to, the alphavirus replicon particles which express TRP2, and any of the TRP2 administrations may be allogeneic or syngeneic with respect to the TRP2 source and the subject.

Further provided is an immunogenic preparation comprising an alphavirus replicon particle which expresses a melanoma antigen dopachrome tautomerase (DCT, TRP2), together with a pharmaceutically acceptable carrier, and optionally additional components to provide an adjuvant and/or a slowed release benefit and/or ingredients to improve storage stability or handling during or after lyophilization such as one or more of a salt, surfactant, bulking agent, plasticizer, and hydrogen-bonding sugar or other polyol.

In the compositions and compositions for use described herein, the alphavirus from which the alphavirus replicon particles are derived can be Venezuelan Equine Encephalitis (VEE) virus, desirably an attenuated VEE virus, which expresses TRP2 in the subject. VEE virus or other alphavirus-derived alphavirus replicon particles (ARPs) can also be engineered for the production of IL-12 or other cytokine. Where there are ARPs expressing IL-12 administered in conjunction with the TRP2-expressing ARP preparation, the dose of the TRP2-expressing VRP is most preferably equal to or greater than the dose of VEE replicon particles expressing IL-12.

### BRIEF DESCRIPTION OF THE DRAWINGS

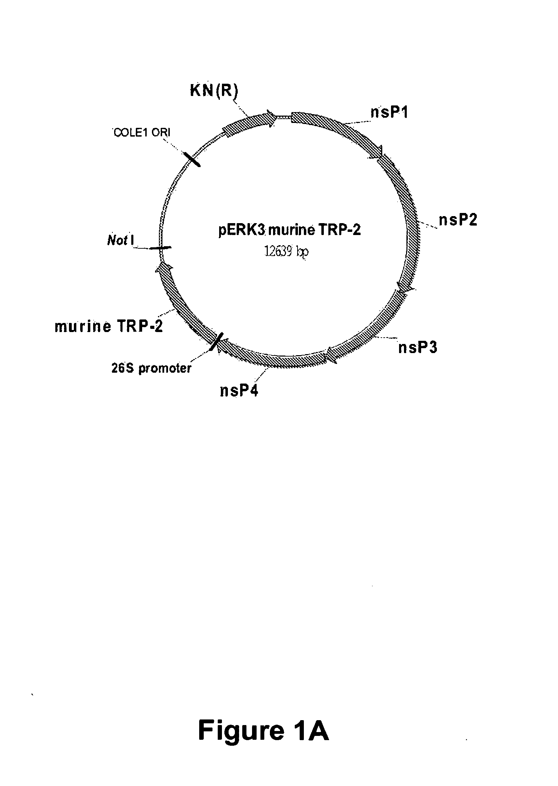

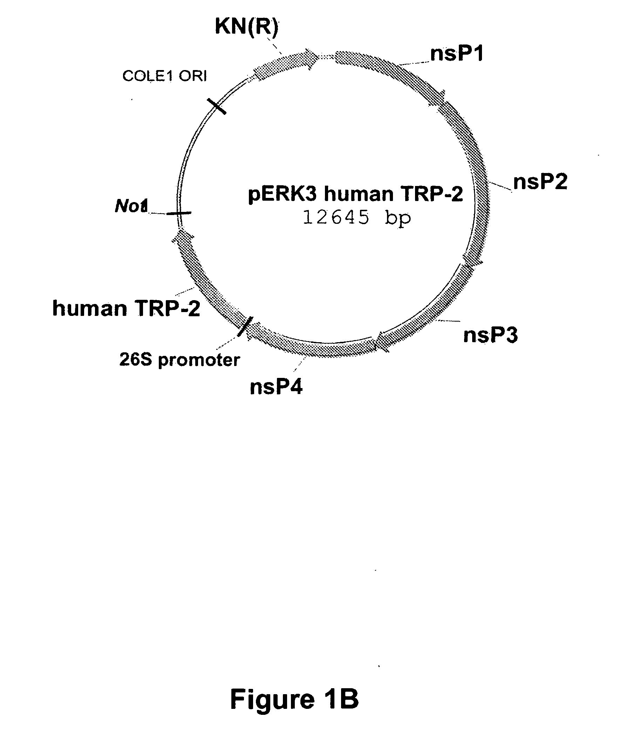

Figure 1A is a diagram of the VEE (mouse) TRP2 replicon. Starting at the T7 promoter and moving clockwise, the solid arrows represent the four VEE viral nonstructural protein genes (nsP1-nsP4), the murine TRP-2 gene and the kanamycin resistance gene (KN^{r}), respectively. Figure 1B is a diagram of the corresponding human construct.

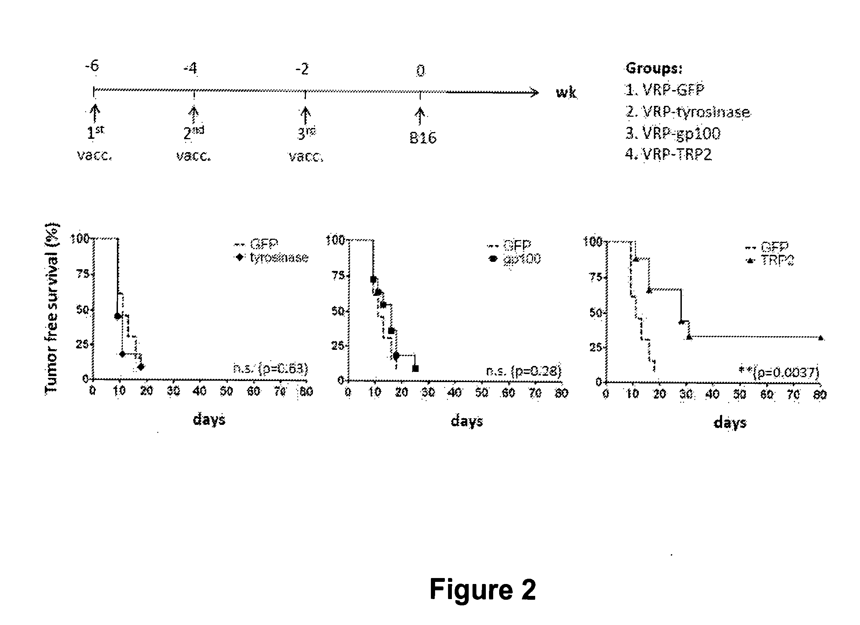

Figure 2 shows the results of an experiment designed to determine which of the indicated melanoma differentiation antigens works best when administered via VRP expression. For this purpose, Groups of 10 mice were vaccinated according to the schedule indicated, and then challenged i.d. with 7.5x10⁴ B16 melanoma cells on the right flank. Tumor growth was monitored every two-three days for 80 days. Tumor free survival is plotted. In two independent experiments we observed that VRP-TRP2 works significantly better than VRP-tyrosinase and VRP-gp100. 30% of the mice treated withVRP-TRP2 were still tumor free 80 days after challenge.

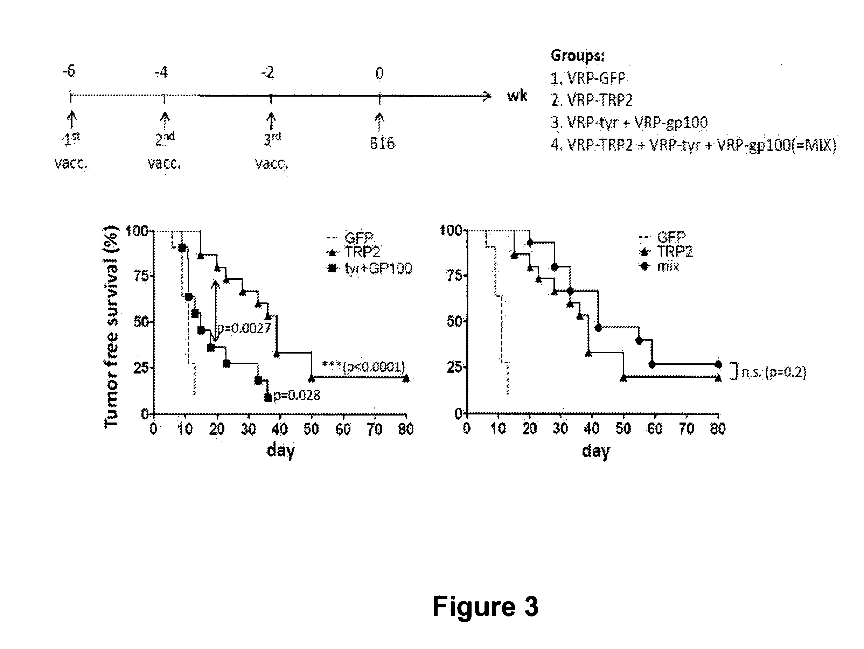

Figure 3 shows the results of an experiment which determined whether combining the three different VRP preparations (VRP-tyr, VRP-gp100, VRP-TRP2) was more effective than using VRP-TRP2 alone. For this purpose, groups of 15 mice were vaccinated as described in the previous figure. Surprisingly, these experiments indicated that, although the combination of VRP-tyrosinase+VRP-gp100 had a significantly better antitumor effect as compared to VRP-GFP, they were not as effective as VRP-TRP2 alone (left panel). Furthermore, the combination of the three different antigens does not increase the efficacy of VRP-TRP2 when administered as a single agent (right panel).

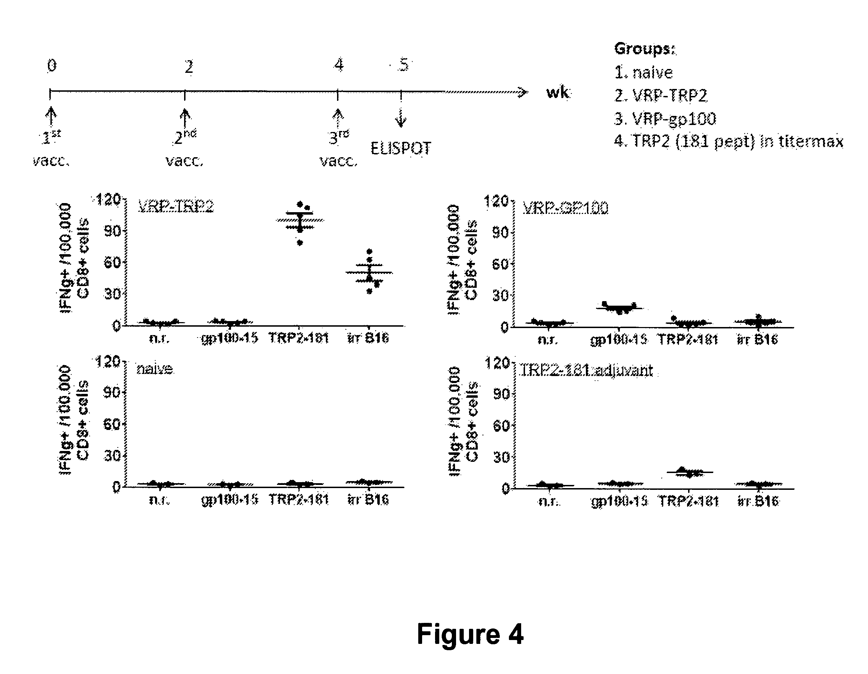

Figure 4 provides the results of a representative experiment where the CD8+T cell response to the vaccine was analyzed. Mice were treated as indicated, with VRP-TRP2, VRP-GP100, the immunodominant TRP2 peptide (TRP2-181) emulsified in titermax (a commercial adjuvant), or left untreated. At week 5 CD8+ T cells were purified from the spleen and re-stimulated with gp100-15 peptide, TRP2-181 peptide, irradiated B16 cells or not re-stimulated (n.r.). IFNγ-secreting CD8+ T cells were analyzed using the ELISPOT assay. Each dot represents an individual mouse; mean and SEM are also indicated. These experiments indicate that VRP-TRP2 can induce CD8+ T cells specific for the 181 epitope and that these CD8+ T cells can recognize B16 cells. VRP-gp100 is also able to activate gp-100 specific CD8+ T cells, but these cells are unable to recognize B16 cells. Tumor specific CD8 T cell responses induced by VRP-TRP2 are significantly higher than those induced by VRP-gp100.

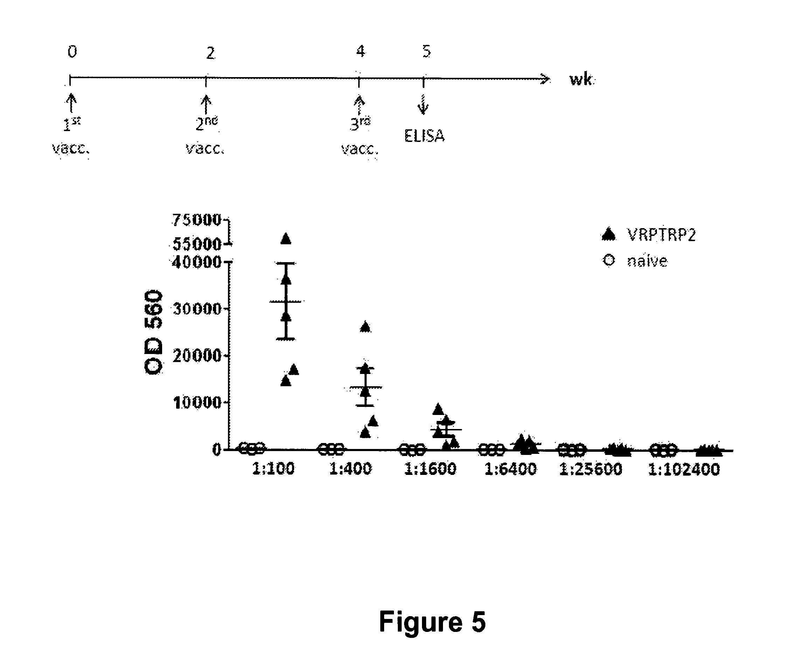

Figure 5 shows the results for analysis of the B cell response induced by the vaccine. Mice were treated as shown, and at week 5, sera were extracted from peripheral blood and analyzed by standard ELISA. On the x-axis is indicated the dilution of the sera (titer). Each symbol represents an individual mouse. This experiment is representative of several VRP-TRP2 induced detectable antibody responses specific for TRP2.

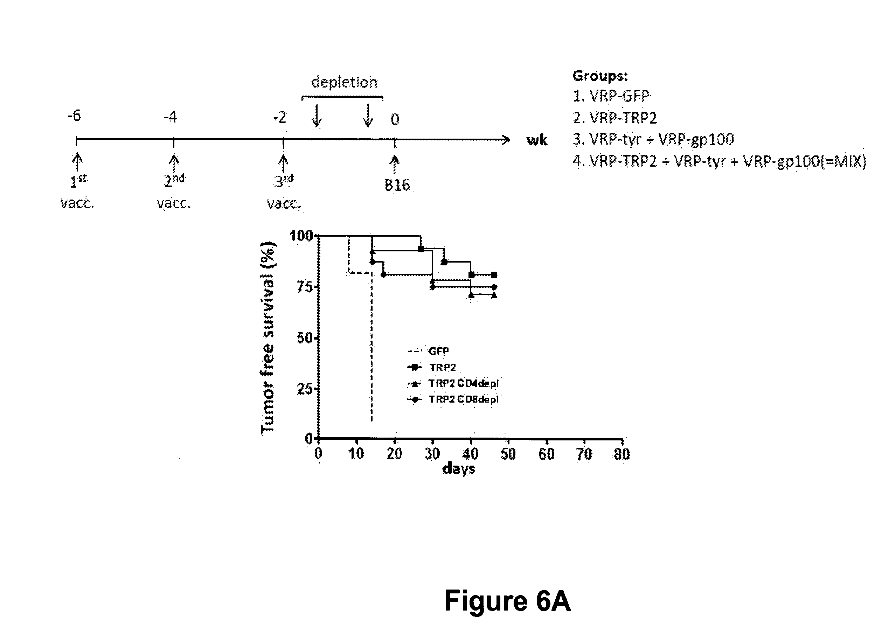

Figure 6A shows results for experiments designed to reveal which arm of the adaptive immune response is more relevant in mediating tumor protection in the prophylactic model. In this experiment mice were vaccinated as per the schedule shown, but after the last immunization and prior to tumor inoculation, CD8+ or CD4+ T cells were depleted by administering depleting antibodies. This experiment (representative of two) suggests that the anti-tumor effect of VRP-TRP2 prophylactic administration does not depend on the CD8+ T cell responses that it is able to induce.

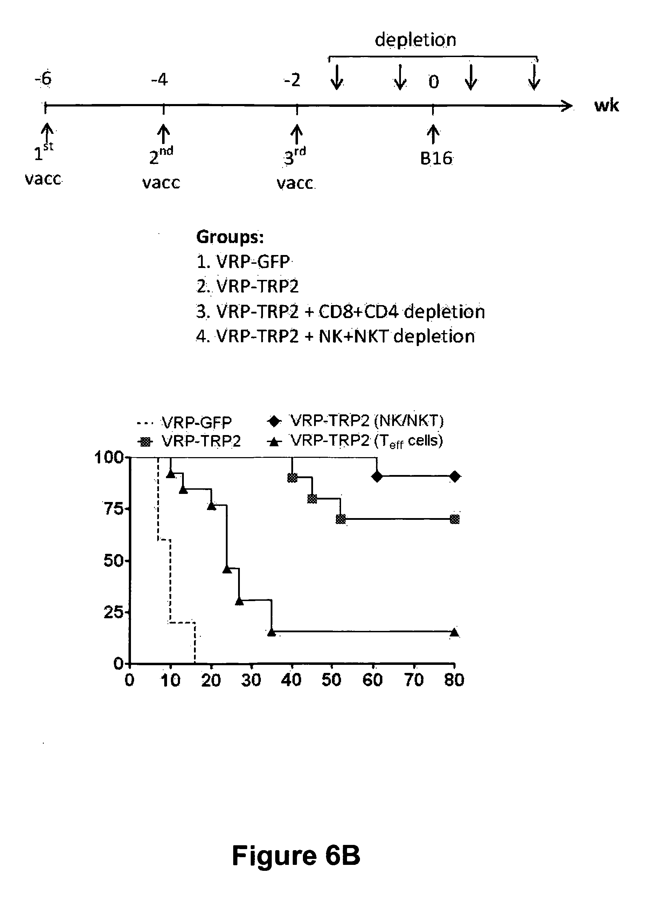

Figure 6B shows the results for further experiments designed to analyze the role of the T- lymphocytes and other immune cells in mediating tumor protection. Mice were vaccinated as per the schedule shown, but after the last immunization, CD8+ and CD4+ T cells, or Natural Killer (NK) and Natural Killer T (NKT) cells, were depleted by administering the appropriate depleting antibodies for an extended period of time, both before and after B16 tumor cell inoculation. Without wishing to be bound by any particular theory, it is believed that this experiment (representative of two) shows that the anti-tumor effect of VRP-TRP2 does not depend on the induction of NK and NKT cells, but that it does, at least in part, depend on the combined action of vaccine-induced CD8+ and CD4+ T cells.

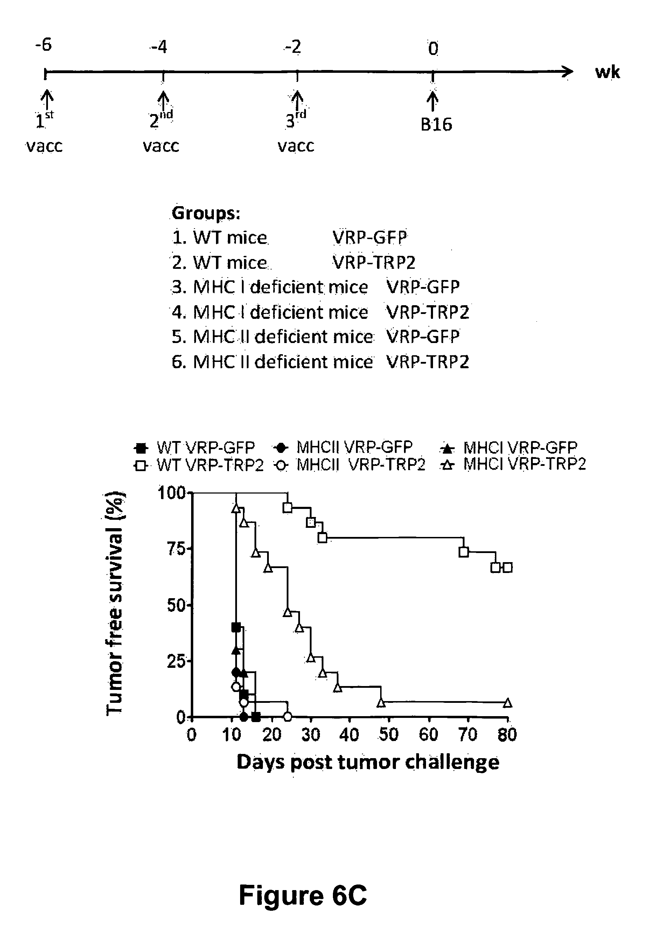

Figure 6C shows the results for additional experiments designed to assess the contribution of CD8+ and CD4+ T lymphocytes and other immune cells in mediating tumor protection. Wild-type (WT), MHC I -deficient, or MHC II-deficient mice (which lack CD4+ T lymphocytes) were vaccinated as per the schedule shown, before the inoculation of B16 tumor cells. Without wishing to be bound by any particular theory, it is believed that MHC II expression is necessary for the anti-tumor effect of VRP-TRP2. By contrast, the lack of MHC I decreases the anti-tumor effect of the vaccine but does not completely eliminate it.

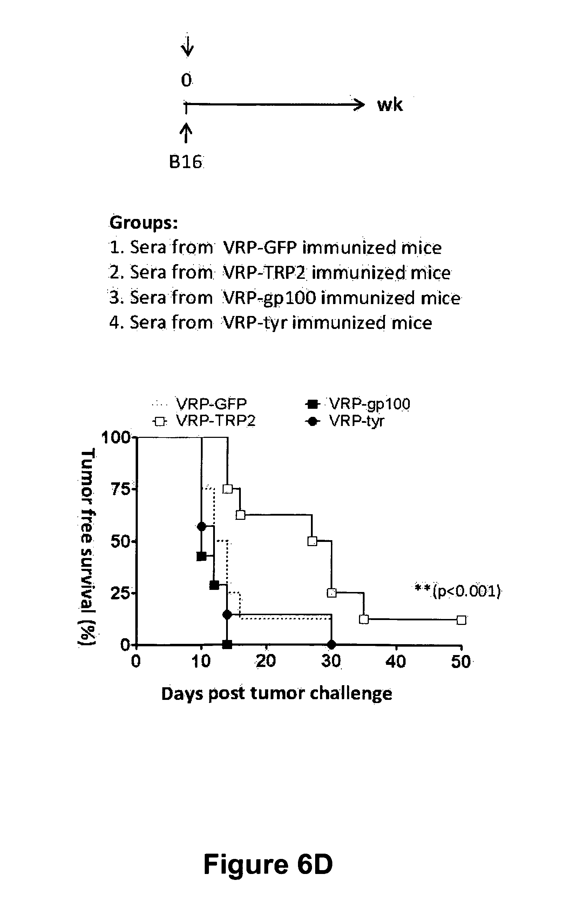

Figure 6D shows the results for experiments designed to reveal the role of IgGs in mediating tumor protection. Groups of mice were vaccinated three times two weeks apart with VRP encoding GFP, TRP2, gp100 or tyr. One week after the last immunization, mice were bled to obtain IgG-containing sera. The sera were then transferred into recipient animals which were inoculated with B16 tumor cells. Without wishing to be bound by any particular theory, it is believed that sera containing anti-TRP2 IgG elicited by the VRP-TRP2 vaccine has anti-tumor properties.

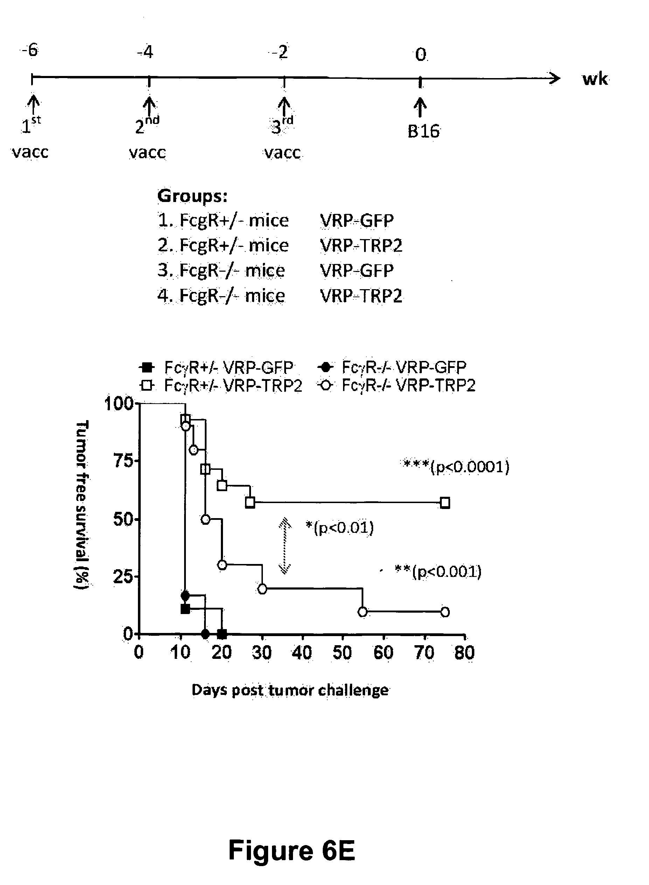

Figure 6E shows additional results for experiments designed to reveal the role of IgGs in mediating tumor protection. Mice deficient in the Fc common gamma chain receptor (FcgR-/-) were vaccinated prior to inoculation with B16 tumor cells. Control littermates were animals that were heterozygous for the deletions were used (FcgR+/-). This experiment indicates that expression of FcgR is an important requirement to achieve a potent anti-tumor effect.

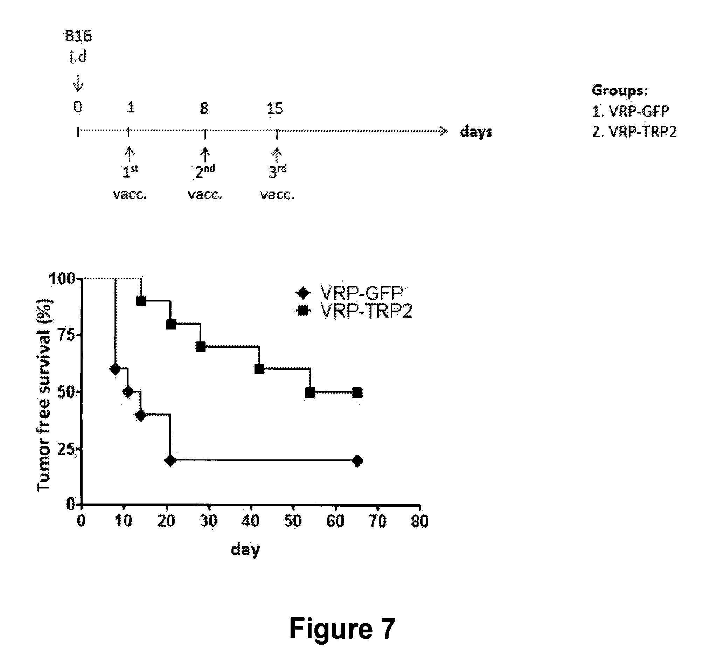

Figure 7 shows the results of experiments designed to evaluate the efficacy of VRP-TRP2 in a therapeutic setting, where injection of the VRPs is started one day after intradermal challenge with B16 melanoma cells (skin model). Mice and tumors are analyzed as described.

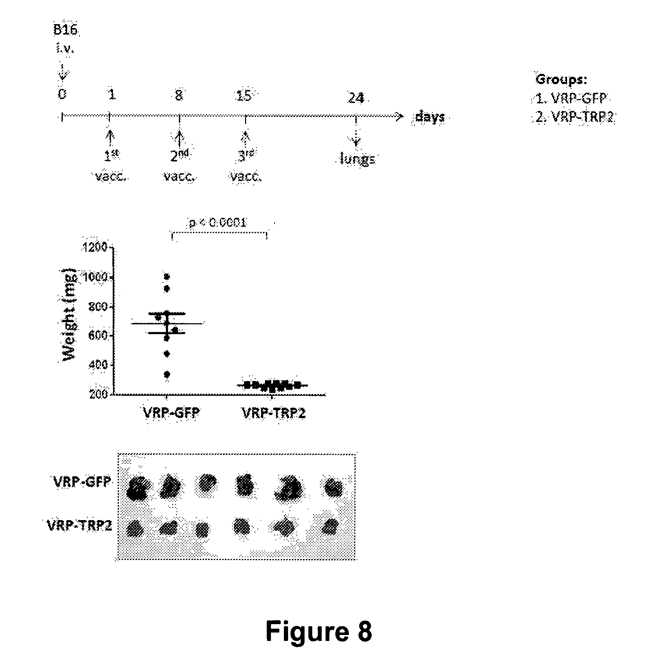

Figure 8 shows the results of an experiment to examine the efficacy of VRP-TRP2 in a treatment model where B16 cells were injected intravenously (i.v.). Following i.v. injection, B16 cells grow primarily in the lung, where they rapidly form nodules (lung model). Typically, 20 to 30 days after B16 cell i.v. injection, mice die with large tumor masses in the lungs. Mice were first challenged i.v. with B16 cells and then treated with VRP-TRP2 as indicated. 24 days after tumor challenge, mice were sacrificed, and lungs analyzed for the presence of lung nodules. 9-10 mice per group were analyzed; lung weight is reported and representative examples are imaged. Mice treated with control VRP-GFP (irrelevant antigen) had very large tumors in the lungs; by contrast VRP-TRP2 treated mice were tumor free or had few very small surface nodules.

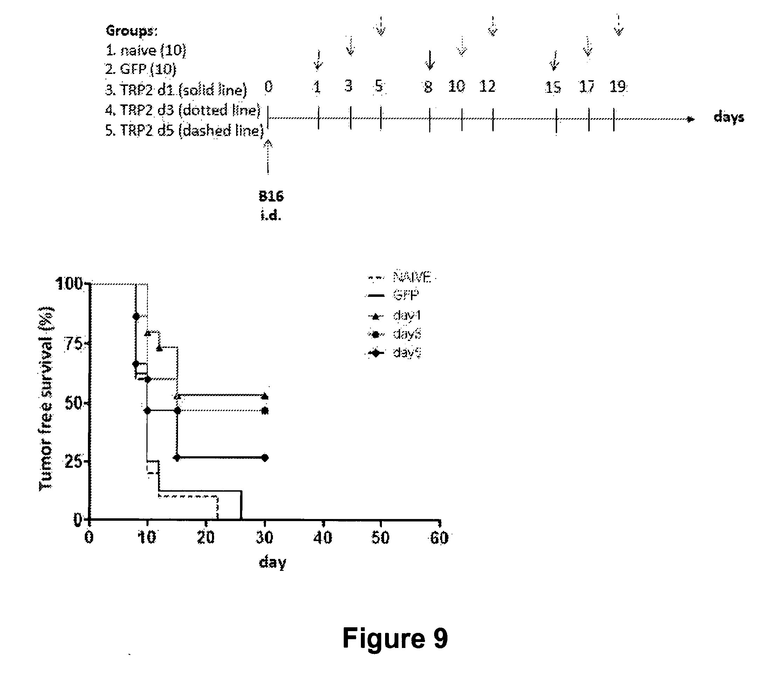

Figure 9 shows the efficacy of VRP-TRP2 in a treatment model where B16 cells were injected intradermally, and treatments were started a different time points after B16 cell injection (skin model, time course). Tumor free survival is reported. Groups of 15 mice were either untreated (naïve), treated with VRP-GFP weekly from day 1, or treated with VRP-TRP2 weekly starting from day 1, day 3 or day 5. This experiment was done to evaluate how late the treatment could be started and still provide a significant anti-tumor effect. Tumor free survival is reported in the chart. Each group of treatment had 10 mice, p= 0.01.

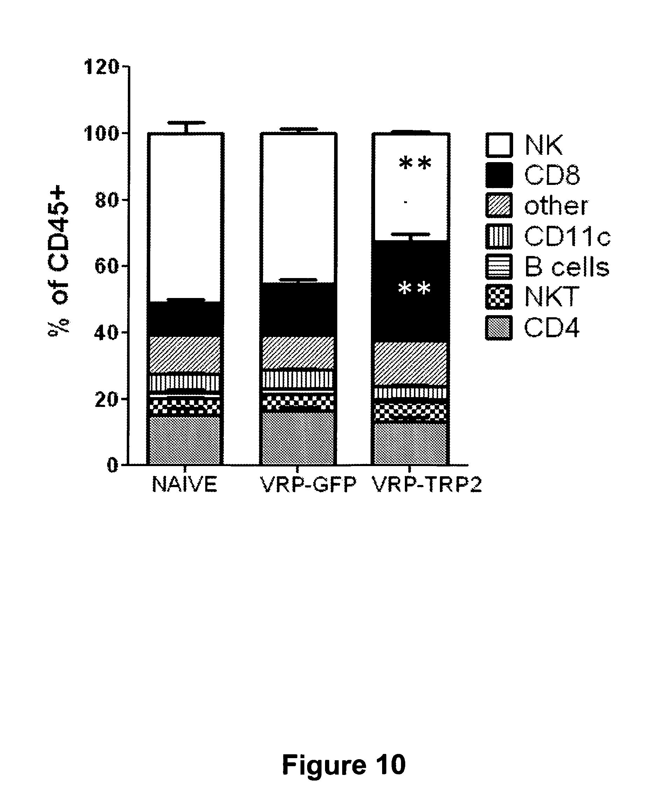

Figure 10 presents the results of an experiment designed to characterize the tumor infiltrate after vaccination with VRP-TRP2, thereby providing an *in vivo* analysis of the immune reaction elicited by the vaccine. B16 tumor cells were inoculated into mice which had been previously vaccinated with the indicated VRP, or left untreated (naïve). Seven days following the B16 tumor cell inoculation, tumor masses were resected and processed for flow cytometric analysis. Panel (a); Representative picture of the tumor masses after resection; Panel (b) Representative dot plots illustrating the flow cytometric strategy utilized for the analysis; Panel (c) Quantification of the indicated population of the types of immune cells infiltrating the tumor (representative of 4-5 mice per group analyzed).

### DETAILED DESCRIPTION OF THE INVENTION

There is a need in the art for cost-effective, potent and efficacious pharmaceutical compositions for the treatment of a devastating cancer such as melanoma and/or for reducing the incidence, metastasis and/or severity of a cancer such as melanoma and for increasing survival time after onset of melanoma. Disclosed herein is an RNA replicon vector system derived from an attenuated alphavirus engineered to produce single-cycle, propagation-defective virus-like alphavirus replicon particle (ARP) containing a self-replicating RNA (replicon) expressing the melanoma antigen dopachrome tautomerase (DCT, also known as TRP2). When inoculated into humans and/or animals, these ARP compositions significantly enhance or induce the humoral and cellular immune responses to melanoma cells, metastatic melanoma cells and tumors. It is particularly important to generate a rapid and strong response to the melanoma cells and tumors in order to increase survival time after diagnosis, delay recurrence, reduce the severity of the cancer and its effects, and/or reduce the intensity and occurrence of this cancer and its effects, which is notoriously difficult to treat, especially if advanced. Additionally, administration of such compositions to subjects not yet having melanoma, e.g., those subjects that may be at high risk for developing melanoma, prevents or reduces the incidence and/or severity of melanoma.

Immunization protocols can have repeated administrations or single administrations, and a second or subsequent dose can be of a different species of TRP2 and or it can comprise a different modality or an additional modality as the initial VRP-TRP2 dosage. Subsequent doses can also be of a different amount, either more or less, than the initial dose administered.

Melanocyte differentiation antigens are attractive candidates for melanoma immunotherapy, but because they are expressed by malignant cells as well as their normal counterpart eliciting effective immune responses is challenging.

The use of an alphavirus replicon vector system as an immunotherapy to prevent or treat melanoma and other TRP2-expressing cancers is described herein. Use of propagation-defective virus-like replicon particles (VRP) based on an attenuated strain of Venezuelan equine encephalitis (VEE) virus is especially attractive because VRP express heterologous proteins to high levels and target expression to dendritic cells. VRP vaccines have been shown to elicit both humoral and cellular immune responses to the vectored gene products in many animal disease models and in phase I/II clinical trials.

The immunogenic protein can be a full-length dopachrome tautomerase (DCT, TRP2) TRP2 protein or an immunogenic fragment thereof. In the alphavirus replicon particle nucleic acid, the TRP2 can be from the same species as that to which the immunogenic composition comprising the alphavirus replicon particles is administered (syngeneic administration) or it can be from a different species (allogeneic administration). A TRP2 protein which varies in sequence from that of the subject but which is derived from the same species as the subject can also be expressed by the VRP composition.

The efficacies of VEE VRP compositions expressing the melanocyte differentiation antigens tyrosinase, gp100 or TRP-2 were analyzed against challenge with cells of the poorly immunogenic and highly aggressive B16 murine melanoma. TRP-2-expressing VRP were the most effective in delaying tumor occurrence when compared to the other antigens tested. Although both TRP2-specific CD8+ T cells and TRP2-specific IgG are induced, the data suggest that B-cells may play a more important role in the effector phase of tumor protection, at least in the prophylactic approach. These studies demonstrate that VRP vaccines present a useful approach for melanoma immunotherapy and prophylaxis.

One aspect of the compositions for use and compositions provided herein is the surprising ability of TRP2 immune response to the melanoma cells, with the result that in the B16 mouse models, there is significant tumor regression and prolonged survival in mice treated after tumor development and postponed or no tumor development in mice challenged with the B16 melanoma cells after vaccination with the TRP2- expressing ARPs. Without wishing to be bound by theory, it is believed that the administration of the TRP2-expressing ARPs enhanced the magnitude of the humoral, or antibody, responses to the melanoma cells as well as the T cell response to those cells. Similar benefit is achieved in humans suffering from melanoma or in humans where protection against melanoma is desired.

The role for the induction of humoral responses by TRP2-expressing ARPs is supported by the observation that sera from mice immunized with TRP2-expressing ARP delayed tumor growth as compared to sera from mice immunized with ARPs expressing other antigens. The induction of TRP-2 specific IgG is dependent on MHC II, and the anti-tumor effect involves signaling through activating Fc receptors.

Immunization with TRP2-expressing ARPs induced a stronger CD8+ T-cell response against the TRP-2₁₈₁ immunodominant epitope as compared to the response elicted by VRPs expressing other melanocyte differentiation antigens. Additionally, immunization with TRP-2-expressing ARPs significantly increased the percentage of infiltrating CD45+ immune cells in the infiltrate of tumors implanted after vaccination, and the quality of the CD45+ response was changed, since the percentage of CD3+CD8+ T cells recruited at the tumor site was significantly enhanced, indicating that the B16 tumor cell-reactive CD8+ T cells were trafficking to the tumor *in vivo.*

Such a synergistic activation of both IgGs and CD8+ T cell effector mechanisms supports the particular efficacy of TRP-2 expressing ARPs.

It is understood that the immunization approach described herein can be combined with surgery and/or chemotherapeutic approaches in the treatment of a diagnosed melanoma.

In certain embodiments, the present methods are practiced as part of a heterologous prime-boost immunization strategy, in which the "priming" immunization, comprising the initial administration of one or more antigens to an animal, especially a human patient, in one form (or "modality") in preparation for subsequent administration(s) (often referred to as "boosting") of the same antigen in a different form, or modality. Specifically, the term "priming", or alternatively "initiating" or "activating" an immune response or "enhancing" and "potentiating", defines a first immunization delivering an antigen which induces an immune response to the desired antigen and recalls a higher level of immune response to the desired antigen upon subsequent re-immunization with the same antigen when administered in the context of a different vaccine delivery system (i.e. form or modality). The forms of antigen to be administered can comprise alphavirus vectors, immunogens derived from cultured or other melanoma cells or of the relevant tumor, for example from the subject if it is a melanoma sufferer, recombinant TRP2 protein, synthetic peptides comprising at least one epitope of TRP2, live, attenuated or killed organisms or extracts where the TRP2 antigen is expressed, naked nucleic acids, nucleic acids formulated with lipid-containing moieties, pox vectors, adenoviral vectors, herpesvirus vectors, flavivirus vectors, vesicular stomatitis virus vectors, paramyxoviral vectors, parvovirus vectors, papovavirus vectors, and retroviral vectors, where a nucleic acid or vector expresses the TRP2 antigen in cells of the subject into which it is administered. The viral vectors can be virus-like particles or viral nucleic acids capable of expressing the TRP2 antigen. In the compositions for use described herein, the priming step is the administration of a composition that comprises the TRP2- expressing ARP. Following the priming immunization a "boosting immunization", or a "boost", is administered, a composition delivering the same antigen as encoded in the priming immunization. The boost is sometimes referred to as an anamnestic response, i.e., an immune response in a previously sensitized animal. Multiple boosts can be administered, utilizing different or the same amounts for each boost. Adjuvants and/or cytokines can be administered together with the immunogenic composition.

Illustrating the need for improvements in prophylaxis and/or treatment of melanoma, over 60,000 Americans are diagnosed with melanoma every year, and this disease causes over 8000 deaths per year (National Cancer Institute, 2008). Melanoma is an especially serious disease, especially if not detected and treated early in the progression of the disease, at least in part because untreated melanoma often metastasizes aggressively. In addition, melanoma often does not respond well to chemotherapeutic regimens.

ARP expressing TRP2 serve as useful immunogens in treatment and/or vaccination regimens to reduce incidence of melanoma and/or to ameliorate subject clinical status or slow the melanoma disease process. In order to determine if ARPs designed to express TRP2 could serve as functional immune-enhancing delivery agents, the coding sequence encoding TRP2 was cloned into the ARP replicon DNA plasmid, and the transcribed RNAs were packaged into VRPs. The procedures used herein for making TRP2-expressing VRP, which are based on a replicon-helper system, are described in detail in U.S. Patent Nos. 7,078,218, 7,045,335, 7,425,337, 7,442,381 and U.S. Published Application 2009-0075384. Mice were immunized with immunogenic compositions containing VRPs which express the TRP2 melanoma antigen. Mice were then monitored for humoral responses to the antigen, and cellular responses were measured in spleens obtained by necropsy performed at the end of the studies. Surprisingly, VRP expressing TRP2 were particularly potent in inducing not only cellular but also humoral responses active against the melanoma tumor cells. No additional antigen type or antibody appeared to be required to generate a beneficial effect in this mouse melanoma model for human disease.

In general, VRP vaccines direct the expression of the gene of interest to the draining lymph node, which also is the site where antigen presentation to naïve T cells occurs. Prior PCR experiments with VRPS expressing a protein of interest have shown that VRP expression of the gene of interest continues for a few days until the expressing cell succumbs. While soluble protein rapidly disappears by degradation as well as diffusion. VRP-mediated expression, on the other hand, is transient and since there is no DNA stage in the alphavirus replicon cycle, there is no risk of integration.

The optimal dose for a given subject and a given disease target is easily determined based on the teachings herein. A range of ARPs expressing TRP2, in a dosage range from 10⁴ to 10¹¹, optionally 10⁷ to 10¹⁰, especially 10⁷, 5 x10⁷,10⁸, 5 x 10⁸, 10⁹, 5 x 10⁹ or 10¹⁰, can be tested. The optimal dose for enhancing cellular responses may not always be the optimal dose for enhancing humoral responses; if both responses are desired, it may be advantageous to test several concentrations of TRP2-ARPs within the range given to determine the optimal overall immune response.

The following discussion is provided to improve the understanding of the present disclosure by one of ordinary skill in the relevant art.

In the context of the present application, nm means nanometer, mL means milliliter, VEE means Venezuelan Equine Encephalitis Virus, EMC means Encephalomyocarditis Virus, BHK means baby hamster kidney cells, HA means hemagglutinin gene, GFP means green fluorescent protein gene, N means nucleocapsid, FACS means fluorescence activated cell sorter, IRES means internal ribosome entry site, pfu means plaque forming units, iu means infectious units, and FBS means Fetal Bovine Serum. The expression "E2 amino acid (e.g., Lys, Thr, etc.) number" indicates designated amino acid at the designated residue of the E2 protein, and is also used to refer to amino acids at specific residues in the E3 or E1 proteins. TRP2 refers to dopachrome tautomerase (also known as the "slaty" locus, DCL or TRP2/DCL).

As used herein, the term "alphavirus" has its conventional meaning in the art, and includes the various species such as VEE Virus, Semliki Forest Virus (SFV), Sindbis, Ross River Virus, Western Equine Encephalitis Virus, Eastern Equine Encephalitis Virus, Chikungunya Virus, S.A. AR86, Everglades Virus, Mucambo Virus, Barmah Forest Virus, Middleburg Virus, Pixuna Virus, O'nyong-nyong Virus, Getah Virus, Sagiyama Virus, Bebaru Virus, Mayaro Virus, Una Virus, Aura Virus, Whataroa Virus, Banbanki Virus, Kyzylagach Virus, Highlands J Virus, Fort Morgan Virus, Ndumu Virus, and Buggy Creek Virus. Alphaviruses useful in the constructs and methods described herein are VEE, S.A. AR86, Sindbis (e.g. TR339, see U.S. Patent No. 6,008,035), and SFV. See also WO 2008/058035 and WO 2008/085557.

The terms "5' alphavirus replication recognition sequence" and "3' alphavirus replication recognition sequence" refer to the sequences found in alphaviruses, or sequences derived therefrom, that are recognized by the nonstructural alphavirus replicase proteins and lead to replication of viral RNA. These are sometimes referred to as the 5' and 3' ends, or alphavirus 5' and 3' sequences. The use of these 5' and 3' ends results in replication of the RNA sequence encoded between the two ends. The 3' alphavirus replication recognition sequence as found in the alphavirus is typically approximately 300 nucleotides in length, which contains a more well defined, minimal 3' replication recognition sequence. The minimal 3' replication recognition sequence, conserved among alphaviruses, is a 19 nucleotide sequence (Hill et al., J. Virology, 2693-2704, 1997). These sequences can be modified by standard molecular biological techniques to further minimize the potential for recombination or to introduce cloning sites, with the proviso that they must be recognized by the alphavirus replication machinery.

The term "minimal 5' alphavirus replication recognition sequence" refers to the minimal sequence that allows recognition by the nonstructural proteins of the alphavirus but does not result in significant packaging/recombination of RNA molecules containing the sequence. In a preferred embodiment, the minimal 5' alphavirus replication recognition sequence results in a fifty to one-hundred fold decrease in the observed frequency of packaging/recombination of the RNA containing that sequence. Packaging/recombination of helpers can be assessed by several methods, e.g. the method described by Lu and Silver (J. Virol. Methods 2001, 91 (1): 59-65).

The terms "alphavirus RNA replicon", "alphavirus replicon RNA" , "alphavirus RNA vector replicon", and "vector replicon RNA" are used interchangeably to refer to an RNA molecule expressing nonstructural protein genes such that it can direct its own replication (amplification) and comprises, at a minimum, 5' and 3' alphavirus replication recognition sequences (which may be the minimal sequences, as defined above, but may alternatively be the entire regions from the alphavirus), coding sequences for alphavirus nonstructural proteins, and a polyadenylation tract. It may additionally contain one or more elements to direct the expression, meaning transcription and translation, of a heterologous RNA sequence. It may also be engineered to express alphavirus structural proteins. Johnston et al., Polo et al. (as cited in the background), and Smith et al. (U.S. Patent Nos. 7,045,335, 7,078,218, 7,425,337 and 7,442,381) describe numerous constructs for such alphavirus RNA replicons. Specific embodiments of the alphavirus RNA replicons may contain one or more attenuating mutations, an attenuating mutation being a nucleotide deletion, addition, or substitution of one or more nucleotide(s), or a mutation that comprises rearrangement or chimeric construction which results in a loss of virulence in a live virus containing the mutation as compared to the appropriate wild-type alphavirus. Examples of an attenuating nucleotide substitution (resulting in an amino acid change in the replicon) include a mutation at nsP1 amino acid position 538, nsP2 amino acid position 96, or nsP2 amino acid position 372 in the alphavirus S.A.AR86, and an example of an attenuating mutation in the noncoding region of the replicon nucleic acid is the substitution of A or C at nucleotide 3 in VEE.

The terms "alphavirus structural protein/protein(s)" refers to one or a combination of the structural proteins encoded by alphaviruses. These are produced by the virus as a polyprotein and are represented generally in the literature as C-E3-E2-6k-E1. E3 and 6k serve as membrane translocation/transport signals for the two glycoproteins, E2 and E1. Thus, use of the term E1 herein can refer to E1, E3-E1, 6k-E1, or E3-6k-E1, and use of the term E2 herein can refer to E2, E3-E2, 6k-E2, or E3-6k-E2. Attenuating mutations can be introduced into any one or more of the alphavirus structural proteins.

The term "helper(s)" or "helper construct(s)", refer to a nucleic acid molecule that is capable of expressing one or more alphavirus structural proteins. Johnston et al., Polo et al. (as cited in the background), Smith et al. (U.S. Patent No. 7,045,335 and 7,078,218) and Kamrud et al. (U.S. Published Application 2009-0075384) describe numerous helper constructs useful for expressing alphavirus structural proteins in the production of ARPs.

The terms "helper cell" and "packaging cell" are used interchangeably herein and refer to the cell in which alphavirus replicon particles are produced. The helper cell comprises a set of helpers that encode one or more alphavirus structural proteins. As disclosed herein, the helpers may be RNA or DNA. The cell can be any cell that is alphavirus-permissive, i.e. cells that are capable of producing alphavirus particles upon introduction of a viral RNA transcript. Alphavirus-permissive cells include, but are not limited to, Vero, baby hamster kidney (BHK), 293, 293T, chicken embryo fibroblast (CEF), and Chinese hamster ovary (CHO) cells. In certain embodiments, the helper or packaging cell may additionally include a heterologous RNA-dependent RNA polymerase and/or a sequence-specific protease. The nucleic acids encoding alphavirus structural proteins can be present in the helper cell transiently or by stable integration into the genome of the helper cell. The nucleic acid encoding the alphavirus structural proteins that are used to produce alphavirus particles can be under the control of constitutive and/or inducible promoters. In one embodiment, the alpha virus structural protein coding sequences can be provided on a single DNA helper (see Smith et al. U.S. Patent No. 7,045,335) or as two helper constructs comprising an IRES element in which the translation of these coding sequences can be controlled by the activity of an IRES element. In such embodiments, the IRES element can be active in the specific helper cell type and not active, or minimally active in other cells types. In particular embodiments, the helper cells comprise nucleic acid sequences encoding the alphavirus structural proteins in a combination and/or amount sufficient to produce an alphavirus particle when a recombinant replicon nucleic acid is introduced into the cell under conditions whereby the alphavirus structural proteins are produced and the recombinant replicon nucleic acid is packaged into alphavirus particles disclosed herein.

The terms "alphavirus replicon particles", "virus replicon particles" or "recombinant alphavirus particles", used interchangeably herein, mean a virion-like structural complex incorporating an alphavirus replicon RNA that expresses one or more heterologous RNA sequences. Typically, the virion-like structural complex includes one or more alphavirus structural proteins embedded in a lipid envelope enclosing a nucleocapsid that in turn encloses the RNA. The lipid envelope is typically derived from the plasma membrane of the cell in which the particles are produced. Preferably, the alphavirus replicon RNA is surrounded by a nucleocapsid structure comprised of the alphavirus capsid protein, and the alphavirus glycoproteins are embedded in the cell-derived lipid envelope. The structural proteins and replicon RNA may be derived from the same or different alphaviruses. In a specific embodiment, the replicon RNA and structural proteins are from VEE, e.g. see Rayner et al., U.S. Patent Publication 2005-0266550. In another embodiment, the replicon RNA is derived from VEE and the structural proteins are derived from Sindbis Virus (see, e.g. Dubensky et al., U.S. Patent No. 6,376,236). The alphavirus replicon particles are infectious but propagation-defective, i.e. the replicon RNA cannot propagate beyond the host cell into which the particles initially infect, in the absence of the helper nucleic acid(s) encoding the alphavirus structural proteins.

A promoter for directing transcription of RNA from DNA, i.e. a DNA dependent RNA polymerase, is employed to produce the alphavirus replicon and helper nucleic acids provided herein. In the present context, a promoter is a sequence of nucleotides recognized by a polymerase and sufficient to cause transcription of an associated (downstream) sequence. In some embodiments, the promoter is constitutive (see below). Alternatively, the promoter may be regulated, i.e., not constitutively acting to cause transcription of the associated sequence. If inducible, there are sequences present which mediate regulation of expression so that the associated sequence is transcribed only when (i) an inducer molecule is present in the medium in or on which the cells are cultivated, or (ii) conditions to which the cells are exposed are changed to be inducing conditions. In the present context, a transcription regulatory sequence includes a promoter sequence and can further include cis-active sequences for regulated expression of an associated sequence in response to environmental signals.

In certain embodiments of replicon and helper RNAs, transcription and translation are controlled separately by different regulatory elements. The replicon contains a promoter that directs transcription; an IRES element; and a coding sequence (e.g. for a heterologous protein or fragment), in which the IRES element is operably located such that translation of the coding sequence is via a cap-independent mechanism directed by the IRES element and not via a cap-dependent mechanism (see U.S. Patent No. 7,442,381 to Smith et al.). The term "transcription" as used herein includes the production of RNA from an alphavirus subgenomic promoter of a recombinant replicon nucleic acid, which can itself be an RNA molecule. That is, the subgenomic promoter on a recombinant replicon or helper RNA molecule can direct the transcription of a messenger RNA encoding a heterologous nucleic acid of interest or an alphavirus structural protein. Separately, the recombinant replicon or helper nucleic acid can be "replicated," i.e., copied from the 5' replication recognition sequence through to the replication recognition sequence.

In RNA helper embodiments and to produce the replicon RNA, a promoter is utilized to synthesize RNA in an *in vitro* transcription reaction, and specific promoters suitable for this use include the SP6, T7, and T3 RNA polymerase promoters. In the DNA helper embodiments, the promoter functions within a cell to direct transcription of RNA. Potential promoters for *in vivo* transcription of the construct include eukaryotic promoters such as RNA polymerase II promoters, RNA polymerase III promoters, or viral promoters such as MMTV and MoSV LTR, SV40 early region, RSV or CMV. Many other suitable mammalian and viral promoters are available in the art. Alternatively, DNA dependent RNA polymerase promoters from bacteria or bacteriophage, e.g. SP6, T7, and T3, may be employed for use *in vivo,* with the matching RNA polymerase being provided to the cell, either via a separate plasmid, RNA vector, or viral vector. In a specific embodiment, the matching RNA polymerase can be stably transformed into a helper cell line under the control of an inducible promoter.

In certain constructs, control of nucleic acid expression at the level of translation is accomplished by introducing an internal ribosome entry site (IRES) downstream of the promoter, e.g. the alphavirus 26S subgenomic promoter, and upstream of the coding sequence, e.g. for the heterologous sequence or an alphavirus structural protein, to be translated. The IRES element is positioned so that it directs translation of the mRNA, thereby minimizing, limiting or preventing initiation of translation of the mRNA from the methyl-7-guanosine (5')pppN structure present at the 5' end of the subgenomic mRNA (the "cap"). These constructs result in the IRES controlling translation of a heterologous sequence independently of promoter-driven transcription. IRESes from many different sources can be employed, including viral IRES elements from picornaviruses, e.g., poliovirus (PV) or the human enterovirus 71, e.g. strains 7423/MS/87 and BrCr thereof; from encephalomyocarditis virus (EMCV); from foot-and-mouth disease virus (FMDV); from flaviviruses, e.g., hepatitis C virus (HCV); from pestiviruses, e.g., classical swine fever virus (CSFV); from retroviruses, e.g., murine leukemia virus (MLV); from lentiviruses, e.g., simian immunodeficiency virus (SIV); from cellular mRNA IRES elements such as those from translation initiation factors, e.g., elF4G or DAP5; from transcription factors, e.g., c-Myc or NF-κB-repressing factor (NRF); from growth factors, e.g., vascular endothelial growth factor (VEGF), fibroblast growth factor (FGF-2) and platelet-derived growth factor B (PDGF B); from homeotic genes, e.g., Antennapedia; from survival proteins, e.g., X-linked inhibitor of apoptosis (XIAP) or Apaf-1; from chaperones, e.g., immunoglobulin heavy-chain binding protein BiP, plant viruses, as well as any other IRES elements now known or later.

Any amino acids which occur in the amino acid sequences referred to in the specification have their usual three- and one-letter abbreviations routinely used in the art: A, Ala, Alanine; C, Cys, Cysteine; D, Asp, Aspartic Acid; E, Glu, Glutamic Acid; F, Phe, Phenylalanine; G, Gly, Glycine; H, His, Histidine; I, Ile, Isoleucine; K, Lys, Lysine; L, Leu, Leucine; M, Met, Methionine; N, Asn, Asparagine; P, Pro, Proline; Q, Gln, Glutamine; R, Arg, Arginine; S, Ser, Serine; T, Thr, Threonine; V, Val, Valine; W, Try, Tryptophan; Y, Tyr, Tyrosine.

As used herein, expression directed by a particular sequence is the transcription of an associated downstream sequence. If appropriate and desired for the associated sequence, there the term expression also encompasses translation (protein synthesis) of the transcribed or introduced RNA. Alternatively, different sequences can be used to direct transcription and translation.

Alphavirus-permissive cells employed in the present methods are cells that, upon transfection with a complete viral RNA transcript, are capable of producing viral particles. Alphaviruses have a broad host range. Examples of suitable packaging cells include, but are not limited to, Vero cells, baby hamster kidney (BHK) cells, chicken embryo fibroblast cells, DF-1, 293, 293T, Chinese Hamster Ovary (CHO) cells, and insect cells.

The phrases "structural protein" or "alphavirus structural protein" as used herein refer to one or more of the alphaviral-encoded proteins which are required for packaging of the RNA replicon, and typically include the capsid protein, E1 glycoprotein, and E2 glycoprotein in the mature alphavirus (certain alphaviruses, such as Semliki Forest Virus, contain an additional protein, E3, in the mature coat). The term "alphavirus structural protein(s)" refers to one or a combination of the structural proteins encoded by alphaviruses. These are synthesized (from the viral genome) as a polyprotein and are represented generally in the literature as C-E3-E2-6k-E1. E3 and 6k serve as membrane translocation/transport signals for the two glycoproteins, E2 and E1. Thus, use of the term E1 herein can refer to E1, E3-E1, 6k-E1, or E3-6k-E1, and use of the term E2 herein can refer to E2, E3-E2, 6k-E2, or E3-6k-E2.

The structural proteins of the alphavirus are distributed among one or more helper nucleic acid molecules (e.g., a first helper RNA (or DNA) and a second helper RNA (or DNA). In addition, one or more structural proteins may be located on the same molecule as the replicon nucleic acid, provided that at least one structural protein is deleted from the replicon RNA such that the replicon and resulting alphavirus particle are replication defective. As used herein, the terms "deleted" or "deletion" mean either total deletion of the specified segment or the deletion of a sufficient portion of the specified segment to render the segment inoperative or nonfunctional, in accordance with standard usage. See, e.g., U.S. Patent No. 4,650,764 to Temin et al. The term "replication defective" as used herein is synonymous with "propagation-defective", and means that the particles produced in a given host cell cannot produce progeny particles in the host cell, due to the absence of the helper function, i.e. the alphavirus structural proteins required for packaging the replicon nucleic acid. However, the replicon nucleic acid is capable of replicating itself and being expressed within the host cell into which it has been introduced.

Methods for the economical and efficient production of high yield particles are described in U.S. Patent No. 7,078,218, issued July 18, 2006, as are specific attenuated strains and viruses useful for the expression of an expressible IL-12 coding sequence. Methods for preparing dried and reconstituted compositions containing VEE-related VRPs are described in WO 2008/058035 and U.S. Published Application 2009/0047255.

The helper cell, also referred to as a packaging cell, used to produce the infectious, replication defective alphavirus particles, must express or be capable of expressing alphavirus structural proteins sufficient to package the replicon nucleic acid. The structural proteins can be produced from a set of RNAs, typically two that are introduced into the helper cell concomitantly with or prior to introduction of the replicon vector. The first helper RNA includes RNA encoding at least one alphavirus structural protein but does not encode all alphavirus structural proteins. The first helper RNA may comprise RNA encoding the alphavirus E1 glycoprotein, but not encoding the alphavirus capsid protein and the alphavirus E2 glycoprotein. Alternatively, the first helper RNA may comprise RNA encoding the alphavirus E2 glycoprotein, but not encoding the alphavirus capsid protein and the alphavirus E1 glycoprotein. In a further embodiment, the first helper RNA may comprise RNA encoding the alphavirus E1 glycoprotein and the alphavirus E2 glycoprotein, but not the alphavirus capsid protein. In a fourth embodiment, the first helper RNA may comprise RNA encoding the alphavirus capsid, but none of the alphavirus glycoproteins. In a fifth embodiment, the first helper RNA may comprise RNA encoding the capsid and one of the glycoproteins, i.e. either E1 or E2, but not both.

In combination with any one of these first helper RNAs, the second helper RNA encodes at least one alphavirus structural protein not encoded by the first helper RNA. For example, where the first helper RNA encodes only the alphavirus E1 glycoprotein, the second helper RNA may encode one or both of the alphavirus capsid protein and the alphavirus E2 glycoprotein. Where the first helper RNA encodes only the alphavirus capsid protein, the second helper RNA may include RNA encoding one or both of the alphavirus glycoproteins. Where the first helper RNA encodes only the alphavirus E2 glycoprotein, the second helper RNA may encode one or both of the alphavirus capsid protein and the alphavirus E1 glycoprotein. Where the first helper RNA encodes both the capsid and alphavirus E1 glycoprotein, the second helper RNA may include RNA encoding one or both of the alphavirus capsid protein and the alphavirus E2 glycoprotein.

In the helper nucleic acids, it is understood that these molecules further comprise sequences necessary for expression (encompassing translation and where appropriate, transcription or replication signals) of the encoded structural protein sequences in the helper cells. Such sequences can include, for example, promoters, (either viral, prokaryotic or eukaryotic, inducible or constitutive), IRES elements, and 5' and 3' viral replicase recognition sequences. Helper nucleic acids with no promoter can also be advantageous (see U.S. Published Application No. 2009-0075384). In the case of the helper nucleic acids expressing one or more glycoproteins, it is understood from the art that these sequences are advantageously expressed with a leader or signal sequence at the N-terminus of the structural protein coding region in the nucleic acid constructs. The leader or signal sequence can be derived from the alphavirus, for example E3 or 6k, or it can be a heterologous sequence such as a tissue plasminogen activator signal peptide or a synthetic sequence. Thus, as an example, a first helper nucleic acid may be an RNA molecule encoding capsid-E3-E1, and the second helper nucleic acid may be an RNA molecule encoding capsid-E3-E2. Alternatively, the first helper RNA can encode capsid alone, and the second helper RNA can encode E3-E2-6k-E1. Additionally, the packaging signal or "encapsidation sequence" that is present in the viral genome is not present in all of the helper nucleic acids. Preferably, the packaging signal(s) are deleted from all of the helper nucleic acids.

These RNA helpers can be introduced into the cells in a number of ways. They can be expressed from one or more expression cassettes that have been stably transformed into the cells, thereby establishing packaging cell lines (see, for example, U.S. Patent No. 6,242,259). Alternatively, the RNAs can be introduced as RNA or DNA molecules that can be expressed in the helper cell without integrating into the cell genome. Methods of introduction include electroporation, viral vectors (e.g. SV40, adenovirus, nodavirus, astrovirus), and lipid-mediated transfection.

An alternative to multiple helper RNAs is the use of a single DNA molecule, which encodes all the polypeptides necessary for packaging the viral replicon RNA into infective alphavirus replicon particles (see U.S. Patent No. 7,045,335). The single DNA helper can be introduced into the packaging cell by any means known to the art, including but not limited to electroporation, lipid-mediated transfection

(lipofection), viral vectored (e.g. adenovirus or SV-40), or calcium phosphate-mediated transfection. Preferably, the DNA is introduced via the electroporation-based methods. The DNA is typically electroporated into cells with a decrease in voltage and an increase in capacitance, as compared to that required for the uptake of RNA. In all electroporation reactions, the value for the voltage and capacitance must be set so as to avoid destroying the ability of the packaging (host) cells to produce infective alphavirus particles. Alternatively, the helper function, in this format and under an inducible promoter, can be incorporated into the packaging cell genome prior to the introduction/expression of the RNA vector replicon, and then induced with the appropriate stimulus just prior to, concomitant with, or after the introduction of the RNA vector replicon.

Advantageously, one or more of the nucleic acids encoding the alphavirus structural proteins, i.e., the capsid, E1 glycoprotein and E2 glycoprotein, or the replicon construct, contains one or more attenuating mutations. The phrases "attenuating mutation" and "attenuating amino acid," as used herein, mean a nucleotide mutation (which may or may not be in a region of the viral genome encoding polypeptides) or an amino acid coded for by a nucleotide mutation, which in the context of a live virus, result in a decreased probability of the alphavirus causing disease in its host (i.e., a loss of virulence), in accordance with standard terminology in the art, See, e.g., B. Davis, et al., Microbiology 156-158, (4th ed. 1990), whether the mutation be a substitution mutation, or an in-frame deletion or addition mutation. The phrase "attenuating mutation" excludes mutations which would be lethal to the virus, unless such a mutation is used in combination with a "restoring" mutation which renders the virus viable, albeit attenuated. Methods for identifying suitable attenuating mutations in the alphavirus genome are known in the art. Olmsted et al. (1984; Science 225:424) describes a method of identifying attenuating mutations in Sindbis virus by selecting for rapid growth in cell culture. Johnston and Smith (1988; Virology 162:437) describe the identification of attenuating mutations in VEE by applying direct selective pressure for accelerated penetration of BHK cells. Attenuating mutations in alphaviruses have been described in the art, e.g. White et al. 2001 J. Virology 75:3706; Kinney et al. 1989 Virology 70:19; Heise et al. 2000 J. Virology 74:4207; Bernard et al 2000 Virology 276:93; Smith et al 2001 J. Virology 75:11196; Heidner and Johnston 1994 J. Virology 68:8064; Klimstra et al. 1999 J. Virology 73:10387; Glasgow et al. 1991 Virology 185:741; Polo and Johnston 1990 J. Virology 64:4438; and Smerdou and Liljestrom 1999 J. Virology 73:1092.

In certain embodiments, the replicon RNA comprises at least one attenuating mutation. In other specific embodiments, the helper nucleic acid(s) include at least one attenuating mutation. In embodiments comprising two helper nucleic acid molecules, at least one molecule includes at least one attenuating mutation, or both can encode at least one attenuating mutation. Alternatively, the helper nucleic acid, or at least one of the first or second helper nucleic acids includes at least two, or multiple, attenuating mutations. Appropriate attenuating mutations depend upon the alphavirus used. For example, when the alphavirus is VEE, suitable attenuating mutations may be selected from the group consisting of codons at E2 amino acid position 76 which specify an attenuating amino acid, preferably lysine, arginine, or histidine as E2 amino acid 76; codons at E2 amino acid position 120 which specify an attenuating amino acid, preferably lysine as E2 amino acid 120; codons at E2 amino acid position 209 which specify an attenuating amino acid, preferably lysine, arginine, or histidine as E2 amino acid 209; codons at E1 amino acid 272 which specify an attenuating mutation, preferably threonine or serine as E1 amino acid 272; codons at E1 amino acid 81 which specify an attenuating mutation, preferably isoleucine or leucine as E1 amino acid 81; and codons at E1 amino acid 253 which specify an attenuating mutation, preferably serine or threonine as E1 amino acid 253. Additional attenuating mutations include deletions or substitution mutations in the cleavage domain between E3 and E2 such that the E3/E2 polyprotein is not cleaved; this mutation in combination with the mutation at E1-253 can be used in the present methods and compositions. Similarly, mutations present in existing live vaccine strains, e.g. strain TC83 (see Kinney et al., 1989, Virology 170: 19-30, particularly the mutation at nucleotide 3), can be used.

Where the alphavirus is the South African Arbovirus No. 86 (S.A. AR86), suitable attenuating mutations may be selected from the group consisting of codons at nsP1 amino acid position 538 which specify an attenuating amino acid, preferably isoleucine as nsP1 amino acid 538; codons at E2 amino acid position 304 which specify an attenuating amino acid, preferably threonine as E2 amino acid position 304; codons at E2 amino acid position 314 which specify an attenuating amino acid, preferably lysine as E2 amino acid 314; codons at E2 amino acid position 376 which specify an attenuating amino acid, preferably alanine as E2 amino acid 376; codons at E2 amino acid position 372 which specify an attenuating amino acid, preferably leucine as E2 amino acid 372; codons at nsP2 amino acid position 96 which specify an attenuating amino acid, preferably glycine as nsP2 amino acid 96; and codons at nsP2 amino acid position 372 which specify an attenuating amino acid, preferably valine as nsP2 amino acid 372. Suitable attenuating mutations useful in embodiments wherein other alphaviruses are employed are known to those skilled in the art.

Attenuating mutations may be introduced into the RNA by performing site-directed mutagenesis on the cDNA which encodes the RNA, in accordance with known procedures. See, Kunkel, Proc. Natl. Acad. Sci. USA 82:488 (1985. Alternatively, mutations may be introduced into the RNA by replacement of homologous restriction fragments in the cDNA which codes for the RNA, in accordance with known procedures, or in cDNA copies using mutagenic polymerase chain reaction methods.

The alphavirus replicon vector is introduced into cells in culture that allow replication of alphaviruses and in which the structural proteins of the alphavirus are also expressed, so that the vector is packaged by the structural proteins into ARPs which are eventually released from the cell. Methods for the preparation of infective, propagation-defective, adjuvant alphavirus replicon particles in high yields are described in U.S. Patent 7,078,218, and formulation methods are described in WO 2008/058035 and U.S. Published Application US 2009/0047255.

As used herein, TRP2 is a protein known to the art. The human melanoma antigen can have amino acid and coding sequences as set forth below. The mouse coding and amino acid sequences are given in Tables 1A and 1B, respectively. Human sequences encoding TRP2 are given in Tables 2A, 3, 4A (see also SEQ ID NOs:3, 4 and 5). Others are known to the art. It is understood that sequences that are significantly similar to those provided herein can be used in place of those provided. Advantageously, substantially similar sequences are 90, 91, 92, 93, 94, 95, 96, 97, 98, 99% identical to the amino acid set forth in SEQ ID NO:6, as determined using sequence comparison methods well known to the art. There may be substitutions of amino acids with similar properties (conservative amino substitutions). For example, glutamate/aspartate; alanine/leucine/isoluicine/methionine/valine; phenylanine/tyrosine/tryptophan; histidine/arginine/asparagine/lysine; and serine/threonine representative similar amino acids. A simple method to calculate percent identity is to align the reference sequence (SEQ ID NO:6) with the query sequence for maximum matching, and treating gaps introduced in either sequence for optimizing alignment as mismatches. Then, the number of matches is divided by total number of residues of SEQ ID NO:6 plus any mismatch (gaps) introduced into SEQ ID NO:6. Advantageously, the sequence of a TRP2 used in a particular regimen of ARP administration has an amino acid sequence substantially the same as that of the species into which the TRP2-expressing ARPs are administered.

It is recognized by those skilled in the art that the coding sequences may vary due to the degeneracy of the genetic code and codon usage. All synonymous sequences which code for the antigen or other polypeptide or protein of interest can be used in the immunization protocols described herein, but proteins with limited variation from a specifically exemplified sequence can also be used within the present methods and compositions. Alternative human sequences and polymorphisms can be found in Frudakis et al. (2003) Genetics 165:2071-2083; Lao et al. (2007) Ann. Human Genet. 71:354-369; Khong and Rosenberg (2002) J. Immunol. 168:951-956;

Additionally, it is recognized by those skilled in the art that allelic variations may occur in the coding sequences which do not significantly change activity of the amino acid sequences of the peptides which those sequences encode. All such immunologically equivalent sequences are included within the scope of this application. It is understood that there may be low levels of nucleotide substitutions within a population of TRP2-expressing ARPs or within the encoded proteins, advantageously less than 15% variation in amino acid sequence from that of a specifically disclosed protein.

Standard techniques for cloning, DNA isolation, amplification and purification, for enzymatic reactions involving DNA ligase, DNA polymerase, restriction endonucleases and the like, and various separation techniques are those known and commonly employed by those skilled in the art. A number of standard techniques are described in such references as Sambrook et al. (1989) Molecular Cloning, Second Edition, Cold Spring Harbor Laboratory, Plainview, New York; Maniatis; Ausubel et al. (2000) Current Protocols in Molecular Biology, Wiley, New York, NY, and other sources well known to the art Abbreviations and nomenclature, where employed, are deemed standard in the field and commonly used in professional journals such as those cited herein.

Pharmaceutical formulations, such as vaccines or other immunogenic compositions, as provided herein, comprise an immunogenic amount of the infectious, propagation defective alphavirus replicon particles or live, attenuated particles in combination with a pharmaceutically acceptable carrier. An "immunogenic amount" is an amount of the infectious alphavirus particles which is sufficient to evoke an immune response in the subject to which the pharmaceutical formulation is administered. An amount of from about 10⁴ to about 10¹¹, optionally 10⁷ to 10¹⁰, especially 10⁷, 5 x 10⁷, 10⁸, 5 x 10⁸, 10⁹, 5 x 10⁹ or 10¹⁰, infectious units, or ARPs per dose is believed suitable, depending upon the age and species of the subject being treated. Exemplary pharmaceutically acceptable carriers include, but are not limited to, sterile pyrogen-free water and sterile pyrogen-free physiological saline solution. Subjects to whom may be administered immunogenic amounts of the infectious, replication defective alphavirus particles include human and animal (e.g., dog, pig, cat, cattle, horse, donkey, mouse, hamster, monkeys, guinea pigs) subjects. Administration may be by any suitable means, such as intraperitoneal, intramuscular, intradermal, intranasal, intravaginal, intrarectal, subcutaneous or intravenous administration.

Immunogenic compositions comprising the ARPs (which direct the expression of the sequence(s) of interest when the compositions are administered to a human or animal) may be formulated by any of the means known in the art. Such compositions, especially vaccines, are typically prepared as injectables, either as liquid solutions or suspensions. Solid forms suitable for solution in, or suspension in, liquid prior to injection may also be prepared. Lyophilized preparations are also suitable, especially as described in WO 2008/058035 and U.S. Published Application US 2009/0047255.

The active immunogenic ingredients (the ARPs) are often mixed with excipients or carriers which are pharmaceutically acceptable and compatible with the active ingredient. Suitable excipients include but are not limited to sterile water, saline, dextrose, glycerol, ethanol, or the like and combinations thereof, as well as stabilizers, e.g. human serum albumin (HSA) or other suitable proteins and reducing sugars

In addition, if desired, the vaccines may contain minor amounts of auxiliary substances such as wetting or emulsifying agents, pH buffering agents, and/or adjuvants which enhance the effectiveness of the vaccine. Examples of adjuvants which may be effective include but are not limited to: aluminum hydroxide; N-acetyl-muramyl-L-threonyl-D-isoglutamine (thr-MDP); N-acetyl-nor-muramyl-L-alanyl-D-isoglutamine (CGP 11637, referred to as nor-MDP); N-acetylmuramyl-L-alanyl-D-isoglutaminyl-L-alanine-2-(1'-2'-dipalmitoyl-sn-glycero-3hydroxyphosphoryloxy)-ethylamine (CGP 19835A, referred to as MTP-PE); and RIBI (which contains three components extracted from bacteria, monophosphoryl lipid A, trehalose dimycolate and cell wall skeleton (MPL+TDM+CWS) in a 2% squalene/Tween 80 emulsion), MF-59 (a sub-micron oil-in-water emulsion of a squalene, polyoxyethylene sorbitan monooleate (Tween^{™} 80) and sorbitan trioleate), and IC31 (from Intercell, a synthetic formulation combining the immunostimulating properties of an antimicrobial peptide, KLK, and an immunostimulatory oligodeoxynucleotide, ODN1a). An immune stimulating protein such as interleukin-12 can also be included, either as a coding sequence co-expressed with the TRP2 on the same or provided in a separate IL-12 expressing VRP. The effectiveness of an adjuvant may be determined by measuring the amount of antibodies and/or T cell response directed against the immunogenic product of the ARP resulting from administration of the immunogen in vaccines which are also comprised of the various adjuvants. Such additional formulations and modes of administration as are known in the art may also be used.

The immunogenic (or otherwise biologically active) ARP-containing compositions are administered in a manner compatible with the dosage formulation, and in such amount as is prophylactically and/or therapeutically effective. The quantity to be administered, which is generally in the range of about 10⁴ to about 10¹¹, or from 10⁷ to 10¹⁰, especially 10⁷ , 5 x 10⁷, 10⁸, 5 x 10⁸, 10⁹, 5 x 10⁹ or 10¹⁰, infectious units per mL in a dose, depends on the subject to be treated, the route by which the ARPs are administered, the immunogenicity of the expression product, the types of effector immune responses desired, and the degree of protection desired. Precise amounts of the active ingredient required to be administered may depend on the judgment of the physician, veterinarian or other health practitioner and may be peculiar to each individual, but such a determination is within the skill of such a practitioner.

The vaccine or other immunogenic composition may be given in a single dose or multiple dose schedules. A multiple dose schedule is one in which a primary course of vaccination may include 1 to 10 or more separate doses, followed by other doses administered at subsequent time intervals as required to maintain and or reinforce the immune response, e.g., weekly, biweekly or at 1 to 4 months for a second dose, and if needed, a subsequent dose(s) after several months/years. For therapeutic vaccination, a multiple dosing regimen of monthly injections or administrations over multiple years may be beneficial.

The references cited in the present disclosure reflect the level of skill in the relevant arts. It is intended that this information can be employed herein, if needed, to exclude (for example, to disclaim) specific embodiments that are in the prior art. For example, when a compound is claimed, it should be understood that compounds known in the prior art, including certain compounds disclosed in the references disclosed herein (particularly in referenced patent documents), are not intended to be included in the claim.

Every formulation or combination of components described or exemplified can be used to practice the invention, unless otherwise stated. The VRP formulations of the present invention can be prepared according to art-known techniques suitable for the relevant particles, especially as described in US Publication 2009/0047255. Specific names of compounds, viruses, genes and proteins are intended to be exemplary, as it is known that one of ordinary skill in the art can name the same compounds differently. When a protein (or the gene encoding it) is described herein such that a particular variant, isoform, alternate splice variant or allele of the compound is not specified, for example, in a formula or in a chemical name, that description is intended to include each allele, isoform and/or variant of the protein or gene individually or in any combination. One of ordinary skill in the art will appreciate that methods, starting materials, synthetic methods, formulations, vectors and additional techniques other than those specifically exemplified can be employed in the practice of the invention without resort to undue experimentation. All art-known functional equivalents, of any such methods, proteins, coding sequences, vectors, starting materials, formulations, and the like are intended to be included in this invention. Whenever a range is given in the specification, for example, a temperature range, a time range, or a composition range, all intermediate ranges and subranges, as well as all individual values included in the ranges given are intended to be included in the disclosure.

As used herein, "comprising" is synonymous with "including," "containing," or "characterized by," and is inclusive or open-ended and does not exclude additional, unrecited elements or method steps unless otherwise described. As used herein, "consisting of" excludes any element, step, or ingredient not specified in the claim element. As used herein, "consisting essentially of" does not exclude materials or steps that do not materially affect the basic and novel characteristics of the claim. Any recitation herein of the term "comprising", particularly in a description of components of a composition or in a description of elements of a device, is understood to encompass those compositions and methods consisting essentially of and consisting of the recited components or elements. The invention illustratively described herein suitably may be practiced in the absence of any element or elements, limitation or limitations which is not specifically disclosed herein.

The terms and expressions herein are meant to be descriptive and not limiting, and there is no intention in the use of such terms and expressions of excluding any equivalents of the features shown and described or portions thereof, but it is recognized that various modifications are possible within the scope of the invention as claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

The exact formulation, route of administration and dosage can be chosen by the individual physician in view of the patient's condition (see e.g. Fingl et. al., in The Pharmacological Basis of Therapeutics, 1975, Ch. 1).

It should be noted that the attending physician would know how to and when to terminate, interrupt, or adjust administration due to toxicity, or to organ dysfunctions, or to debilitation of the subject. Conversely, the attending physician would also know to adjust treatment to higher levels if the clinical response were not adequate (precluding toxicity). The magnitude of an administered dose in the management of a TRP2-expressing cancer, such as melanoma, will vary with the severity of the condition to be treated and to the route of administration. The severity of the condition may, for example, be evaluated, in part, by standard prognostic evaluation methods. Further, the dose and optionally, dose frequency will also vary according to the age, body weight, and response of the individual patient. A program comparable to that discussed above also may be used in veterinary medicine.

Where the subject is a melanoma sufferer, the physician (or veterinarian in the case of an animal subject) may elect a comprehensive treatment strategy that combines the administration of the immunogenic TRP2-expressing VRP compositions with additional treatments (which may be administered prior to, at the same time as, or after VRP administration), such as radioactive or chemical chemotherapeutic agents, monoclonal antibodies specific for one or more cancer-specific antigens, e.g., CTLA4 or PD1, or melanoma antigens such as TRP2, interleukins or conjugates of the foregoing, or with surgical intervention. Immunosuppressive drugs may be used, e.g. paclitaxel or carboplatin as an initial treatment for the cancer, and after their effect has waned, TRP2-expressing VRP compositions may be given.

Depending on the specific condition of the subject being treated or prophylactically vaccinated, the compositions described herein may be formulated and administered systemically or locally. Techniques for formulation and administration may be found in various art references well known and readily accessible to the art. Suitable routes of administration may include, for example, oral, rectal, transdermal, vaginal, transmucosal, intestinal, intramuscular, subcutaneous, intradermal, intramedullary, intrathecal, intravenous, or intraperitoneal injections. For example, for prophylactic applications, the immunogenic compositions comprising as the active immunogenic agent the TRP2-expressing VRPs are generally administered via a subcutaneous or intramuscular route. In the case of the subject with metastatic melanoma, an intravenous route may be chosen, or the subcutaneous or intramuscular route may be selected. There can also be simultaneous administration via multiple routes.

For injection, the immunogenic compositions of the present invention may be formulated in aqueous solutions, preferably in physiologically compatible buffers such as Hanks' solution, Ringer's solution, or physiological saline buffer. For transmucosal administration, penetrants as generally known in the art and appropriate to the barrier to be permeated are used in the formulation.

Use of pharmaceutically acceptable carriers to formulate the compounds herein disclosed for the practice of the invention into dosages suitable for systemic administration is within the scope of the invention. With proper choice of carrier and suitable manufacturing practice, the compositions of the present invention, in particular those formulated as solutions, may be administered parenterally, such as by intravenous injection. Appropriate compounds can be formulated readily using pharmaceutically acceptable carriers well known in the art into dosages suitable for oral administration.

Pharmaceutical compositions suitable for use in the present invention include compositions wherein the active ingredients are contained in an effective amount to achieve the intended purpose. Determination of the effective amounts is well within the capability of those skilled in the art, especially in light of the detailed disclosure provided herein.

The pharmaceutical compositions of the present invention may be manufactured in a manner that is itself known, e.g., by means of conventional mixing, dissolving, granulating, levitating, emulsifying, encapsulating, entrapping or lyophilizing processes.

Pharmaceutical preparations for use in the present methods can be obtained by combining the immunologically active TRP2-expressing VRPs with solid excipient, but liquid preparations can be prepared as described in U.S. Patent Publication 2009/0047255 can be prepared and then lyophilized for reconstitution prior to administration to the subject. Suitable excipients are, in particular, fillers such as sugars, including lactose, sucrose, mannitol, or sorbitol; cellulose preparations such as, for example, hydroxethyl starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate, plasticizers, surfactants and other agents suitable for administration in immunogenic compositions to humans and/or animals. Immunological adjuvants and/or cytokines which enhance an immune response can also be included in these compositions.

In general the terms and phrases used herein have their art-recognized meaning, which can be found by reference to standard texts, journal references and contexts known to those skilled in the art. The following definitions are provided to clarify their specific use in the context of the invention.

Although the description herein contains certain specific information and examples, these should not be construed as limiting the scope of the invention as claimed but as merely providing illustrations of some of the possible embodiments of the invention. For example, thus the scope of the invention should be determined by the appended claims and their equivalents, rather than by the examples given, but the invention may be further understood by the non-limiting examples given herein above.

### Murine TRP-2 cloning

The full length murine TRP-2 gene was PCR amplified from a DNA plasmid obtained from the NIH. Primers for PCR amplification of this plasmid are given below:
murine TRP-2 EcoR5 forward primer (SEQ ID NO:7)
   5'-GGGACTTGTAGGATGGGGACTTTTGTTGGGATGTTTGGG-3'
murine TRP-2 Xba reverse primer (SEQ ID NO:8)
   5'-GGATGGCTCTAGATTAATTAATTATCATGCTTCCTCGGTATATC-3'

The resulting coding sequence for the TRP2 protein is given in Table 1 B and the encoded protein in Table 1 A.

### Replicon Production

This TRP2 product was digested with Pacl and ligated into the pERK3 replicon vector to generate pERK3-murine TRP-2. The pERK3 VEE replicon vector is derived from the pERK plasmid described in U.S. Patent No. 6,783,939, Example 2. pERK3 has two additional restriction endonuclease cleavage sites, Scal and SnaBII in the multiple cloning sequence (MCS), the VEE 5' end, the VEE nonstructural proteins 1-4, the VEE 26S promoter, a multiple cloning site, and the VEE 3' UTR (untranslated region).

### VRP Production

The TRP2-expressing replicon shown in Figure 1A or 1B is packaged using a bipartite RNA helper system, which has been described in US Patent Nos. 5,792,462, US 6,783,939 (Olmsted, et al.), US 7,045,335 (Smith et al.), and US 7,078,218).

Vero cells were expanded in EMEM + 5% FBS to p146, trypsinized, washed and resuspended in 5% sucrose/60mM NaCl/10mM NaPi buffer pH 7.3 to a concentration of 1.2 x 10⁸ cells/mL. 0.5 mL of cells were mixed with murine TRP-2 replicon, capsid helper (dHcap4, as described in U.S. Patent No. 7,045,335) and glycoprotein (3.4.16; also referred to as GP 3014), helper RNAs at an RNA ratio of replicon 30 µg:capsid helper 30 µg:GP helper 60 µg. The glycoprotein helper is fully described, including its nucleic acid sequence, in U.S. Patent Number 6,783,939. The capsid helper is fully described in U.S. Patent No. 7,045,335 (Smith et al.). The mixture was transferred into 0.4 cm gap cuvettes and electroporated at 580 volts, 25 µFD for a total of 4 pulses. Electroporation mixes were held at room temperature for approximately 10 min and then seeded into roller bottles containing 100 mL of OptiPro growth media with 4 mM glutamine. Murine TRP-2 VRP were harvested from cells by a high-salt elution (salt wash, see U.S. Patent No. 7,078,218) process with a yield of 250 IU/cell. VRP were purified by Cellufine sulfate chromatography, formulated into 1% mouse serum albumin (MSA), 4% sucrose, 30 mM sodium gluconate, 500 mM sodium chloride, 10 mM sodium phosphate pH 7.3 and stored at -80°C. VRP were titered with VEE nsP2 antibody.

### Human TRP-2 Viral Replicon Particles

First, the full length human TRP-2 gene is amplified from plasmid DNA. The amplification products are digested with Pacl and ligated into the pERK3 replicon vector similarly cut with Pacl to generate pERK3-humanTRP-2.
Primers for PCR amplification
human TRP-2 EcoR5 forward primer
5'-GAGCCCCCTTTGGTGGGGGTTTCTGCTCAGTTGCTTGG -3'
human TRP-2 Xba/Pacl reverse primer
5'-GGATGGCTCTAGATTAATTAATTACTAGGCTTCTTCTGTGTATC-3'

Starting at the T7 promoter and moving clockwise in Fig. 1B, the solid arrows represent the four VEE viral nonstructural protein genes (nsP1-nsP4), the human TRP-2 gene and the kanamycin resistance gene [KN (R)], respectively.

Human TRP2-expressing VRPs are prepared as described above but formulated with human serum albumin rather than MSA. In general, VRPs are formulated with serum albumin derived from the same species as that to which the preparation is to be administered.

### VRP Immunization and Tumor Challenge

### Mice and cell lines.

C57BL/6J (8-10-wk-old females) and C3 deficient mice (strain B6.129S4-C3tm1 crr/J) were obtained from the Jackson Laboratory (Bar Harbor, ME). MHC class I deficient (strain B2MN12), MHC class II deficient (strain ABBN12) and wild-type (WT) controls mice (strain B6NTac) were purchased from Taconic Farms, Inc. (Hudson, NY). Mice deficient in the FcR common g chain, provided by J. Ravetch (Rockefeller University, New York, NY) were backcrossed onto wild type C57BL/6 and bred at MSKCC.

The B16-F10 mouse melanoma line was originally obtained from I. Fidler (M.D. Anderson Cancer Center, Houston, TX), B78H1 (a Tyrp-1 and TRP-2 negative mouse melanoma cell line derived from B16) and the Lewis Lung carcinoma were cultured in complete RPMI. Unless otherwise noted, mice were vaccinated three times with 10⁶ VRP diluted in PBS by subcutaneous injection into the plantar surface of each footpad for each vaccination.

In the (prophylactic) tumor protection experiments, C57BL/6 mice were vaccinated three times, at fourteen day intervals, with 10⁶ VRP diluted in PBS by subcutaneous injection into the plantar surface of each footpad. In a subset of these mice, individual mice were challenged with 7.5 x 10⁴ B16F10 melanoma cells injected intradermally (i.d.) two weeks after the third bi-weekly vaccination with VRPs.

In the therapeutic tumor protection experiments, C57BL/6 mice were first injected with 7.5 x 10⁴ B16F10 cells either intradermally (cutaneous therapeutic model) or intravenously (lung therapeutic model), and then vaccinated weekly for three times, beginning one day after the challenge. Following vaccination, tumor diameters were measured with calipers; in some experiments, animals were sacrificed when tumors reached 1 cm diameter, ulcerated or caused discomfort to the animals. For other studies, animals were monitored every 2 to 3 days for 80 days. For the assessment of lung tumor development, lungs were collected 24 days after the intravenous tumor cell challenge, washed three times with PBS and weighed. Tumor-free survival is reported in the figures herein. Each group of mice had 10 mice, p+0.01. For the Kaplan-Meier tumor-free survival curves, mice were considered tumor-free until tumors were visible or palpable P values are calculated with Log-Rank (Mantel-Cox) test.

In the T cell depletion experiments (Figure 6B), CD4+ cells, CD8+ cells and NK/NKT cells were depleted by injecting 200µg of GK1.5, 2.43 and PK136 antibodies, respectively, intraperitoneally at day -11, -4, +4, +11, relative to tumor inoculation.

### ELISPOT and T-Cell Assays

Peptides analyzed, including TRP2₁₈₁₋₁₈₉ were synthesized by Genemed Synthesis (San Antonio, TX) and were greater than 80% purity, as determined by high performance liquid chromatography.

Spleens harvested 5 to 7 days after the third VRP immunization were mechanically disrupted, and RBCs were lysed. CD8+ T cells were positively selected from this mixture by incubation with magnetic anti-CD8 beads (Miltenyi Biotec, Bergisch Gladbach, Germany). Interferon γ (IFN-γ) production was determined using the standard ELISPOT assay after 20 to 36 h of incubation of CD8+ T cells (10⁵ per well) with splenocytes pulsed with 1 µg/mL of the noted TRP2-specific peptide. Plates were analyzed using an automated ELISPOT reader system with KS 4.3 software (Carl Zeiss, Thomwood, NY). Flow cytometry based intracellular staining assays were performed on CD8⁺ T cells cultured as above, then incubated overnight with monensin prior to staining with the fixation and permeabilization kit (eBioscience, San Diego, CA) using anti-CD8-PE-Texas red, anti-CD3-FITC, anti-IFNγ-APC (BD Biosciences, San Jose, CA) and LIVE/DEAD Fixable Aqua Dead Cell Stain kit (ViD) (Invitrogen, Carlsbad, CA).

### ELISA

To detect TRP2-specific total IgGs circulating in sera, groups of mice were immunized with TRP2-expressing VRPs three times, with each immunization two weeks apart. Seven days to two weeks after the last vaccination, mice were bled from the tail vein. Blood samples were incubated at 37° C for an hour and centrifuged for 5 min at 7000g. The pooled serum was then collected in a separate tube and stored at -20° C.

ELISA plates were (Costar) were coated O/N with 1 µg/ml of purified recombinant TRP2 in PBS, blocked with 5% Nonfat reconstituted dry milk in PBS for 2 hours, and incubated at 4° C with serial dilutions of serum (4 fold: 1:100, 1:400, 1:1600, etc.) in blocking solution. After 18 hours, plates were washed 6 times with PBS+0.1%Tween 20 and incubated with the second antibody (goat anti mouse IgG-AP, Southern Biotech, Birmingham, AL) in blocking buffer for 1 hour at room temperature. Plates were then washed and incubated in the dark for 30 min with the substrate (Promega, Madison, WI, S1013), and color development was stopped by adding 3N NaOH. Plates were analyzed using an ELISA reader at Excitation 450/50 and Emission 580/50 with gain of 25 (Perseptive Biosystems, Framingham, MA, Cytoflour Series 4000).

### Analysis of the tumor infiltrate

Seven days after B16-Matrigel subcutaneous injection (1 x 10⁵ B16-F10 cells in 0.2 ml of Matrigel Matrix Growth Factor Reduced; BD Biosciences), the matrigel plug was resected, incubated for 1 hour at 37°C with 1 mg/ml Collagenase D (Sigma-Aldrich, St. Louis, MO) and dissociated to obtain a single-cell suspension. Cells were stained with anti-CD45.2 PercpCy5.5, anti-CD3-FITC, anti-NK1.1-APC, anti-CD8-PE-Texas red, anti-CD4- Alexa Fluor 700 (Becton Dickinson) and DAPI.

### Serum transfer

Serum samples were collected from mice seven days after the third bi-weekly VRP immunization. Fifty µl of serum was injected i.v. in recipient mice at day 0, 3 and 6. Recipient mice were then challenged with 7.5 x 10⁴ B16-F10 tumor cells intradermally at day 0, and tumor occurrence was monitored as described above.

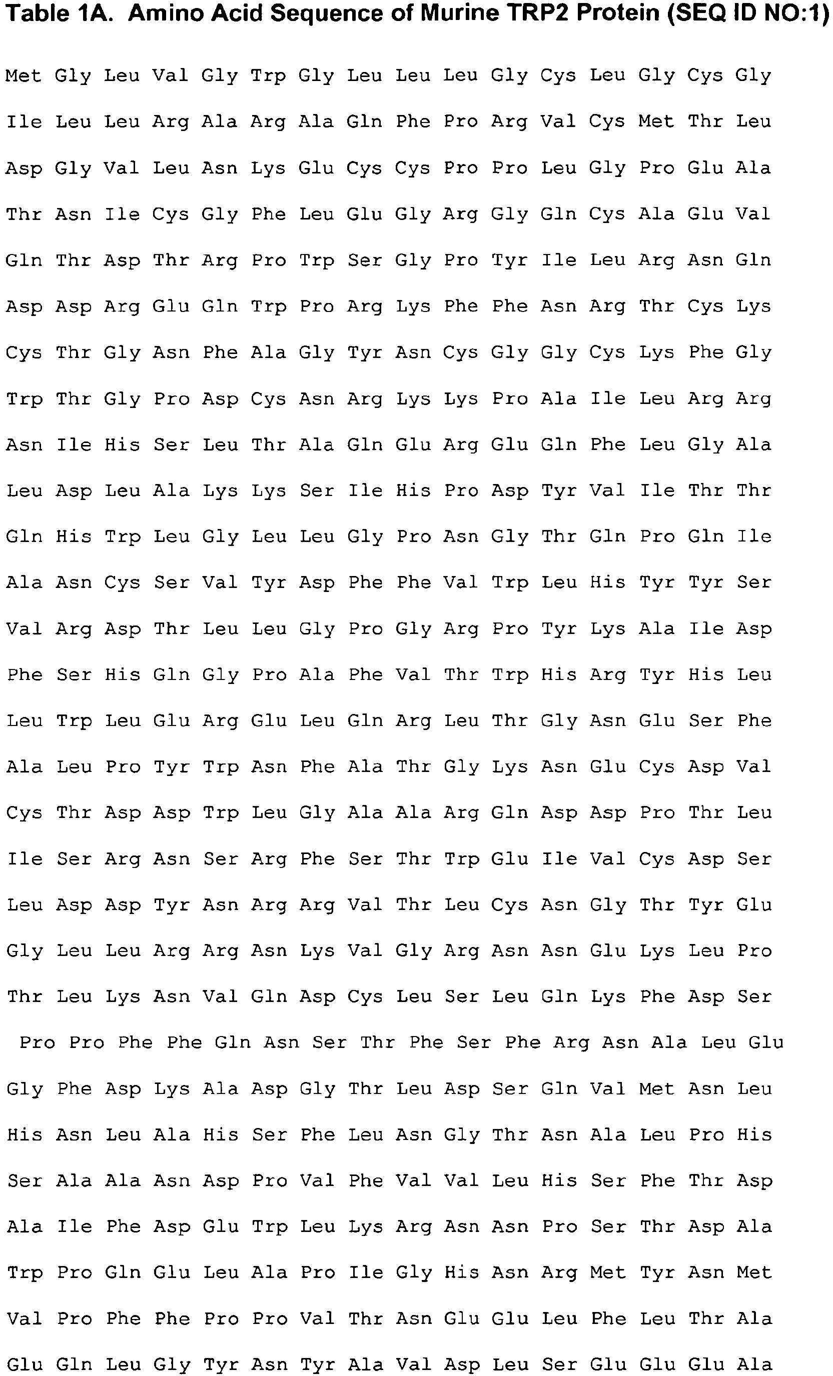

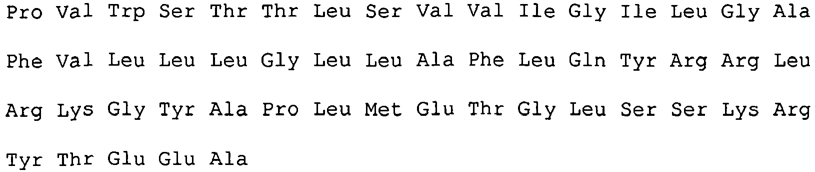

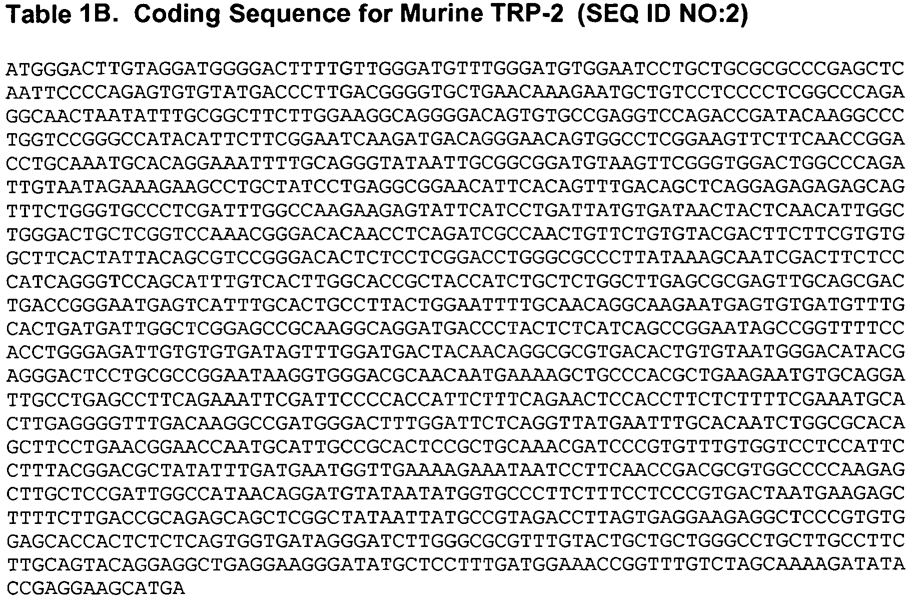

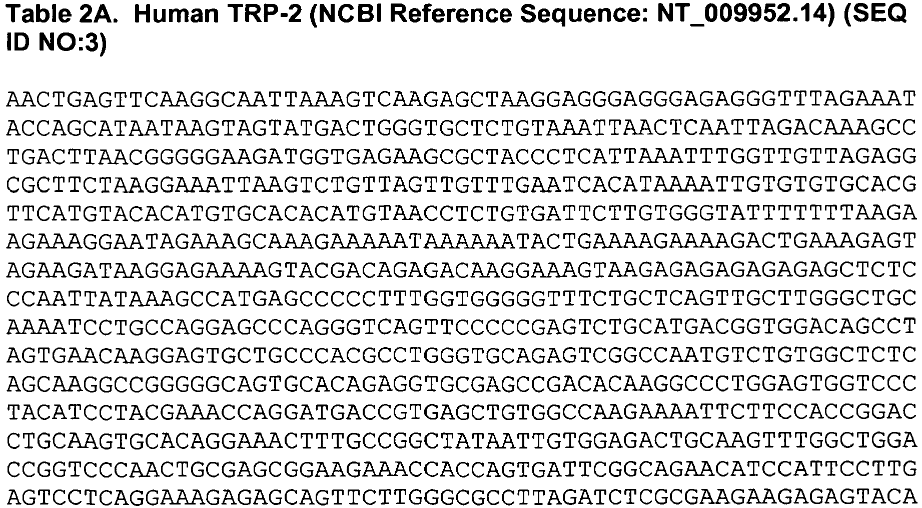

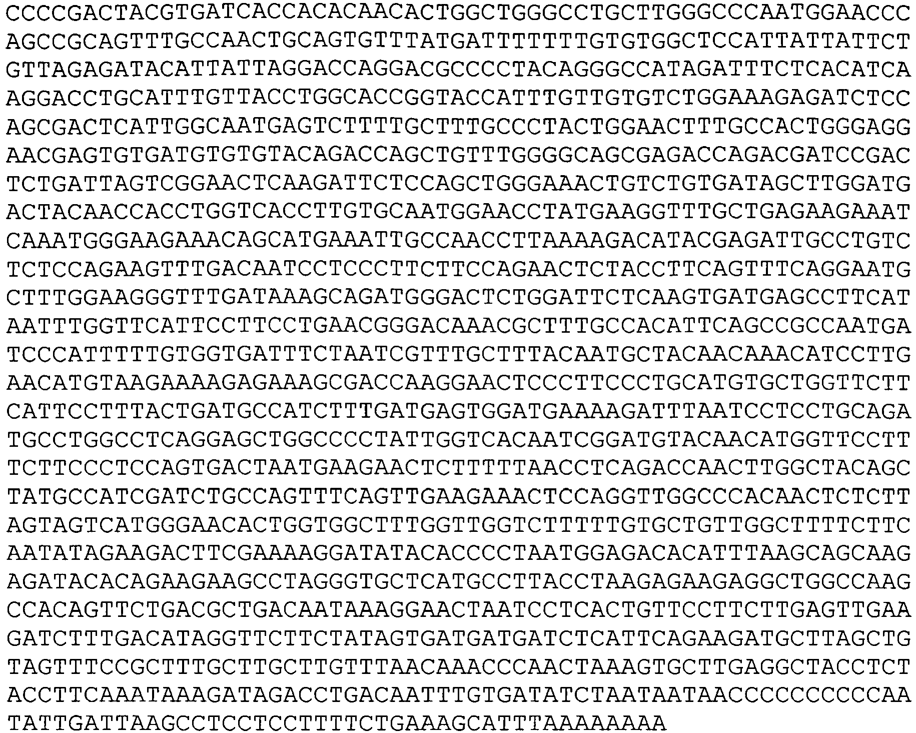

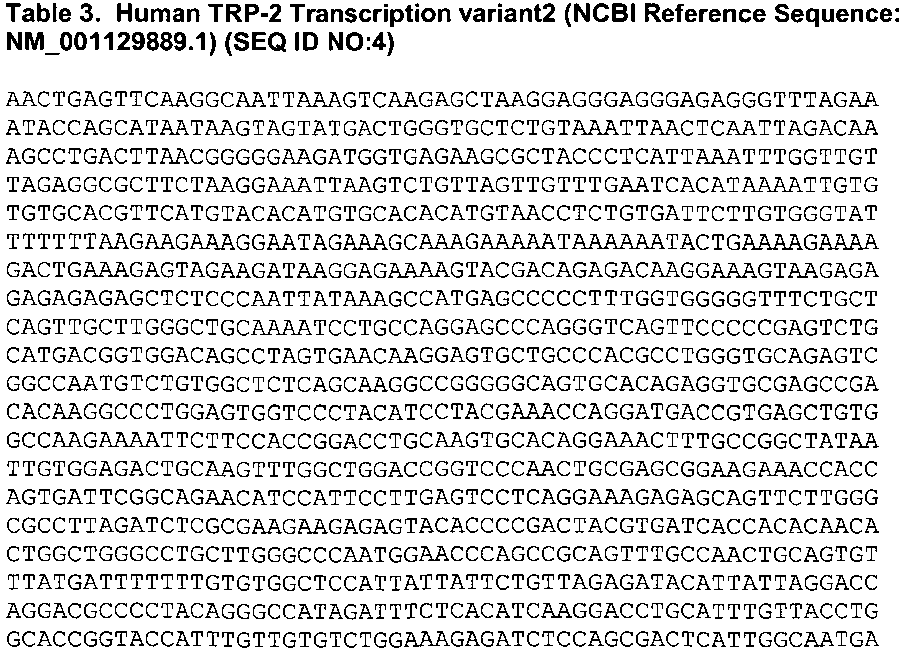

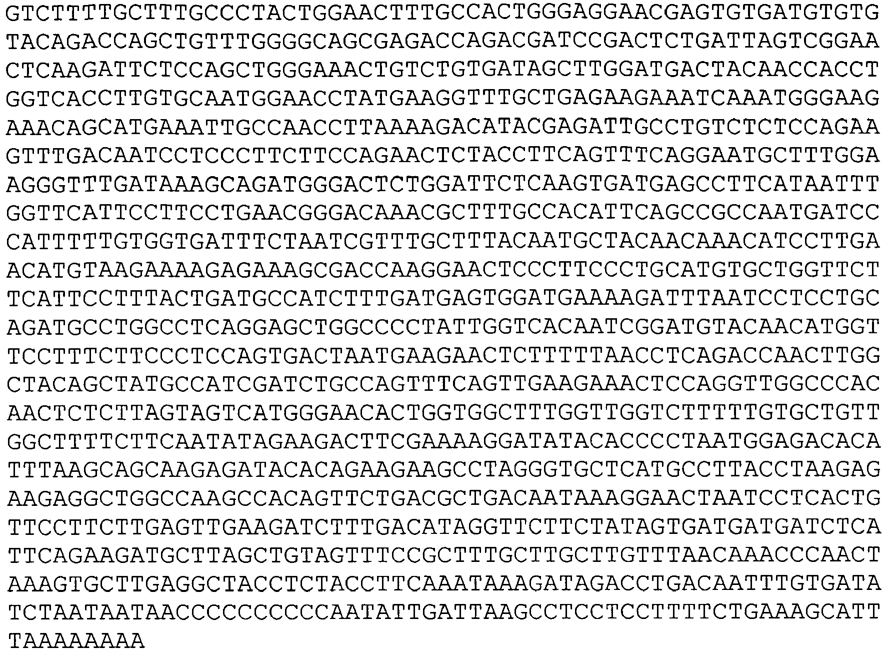

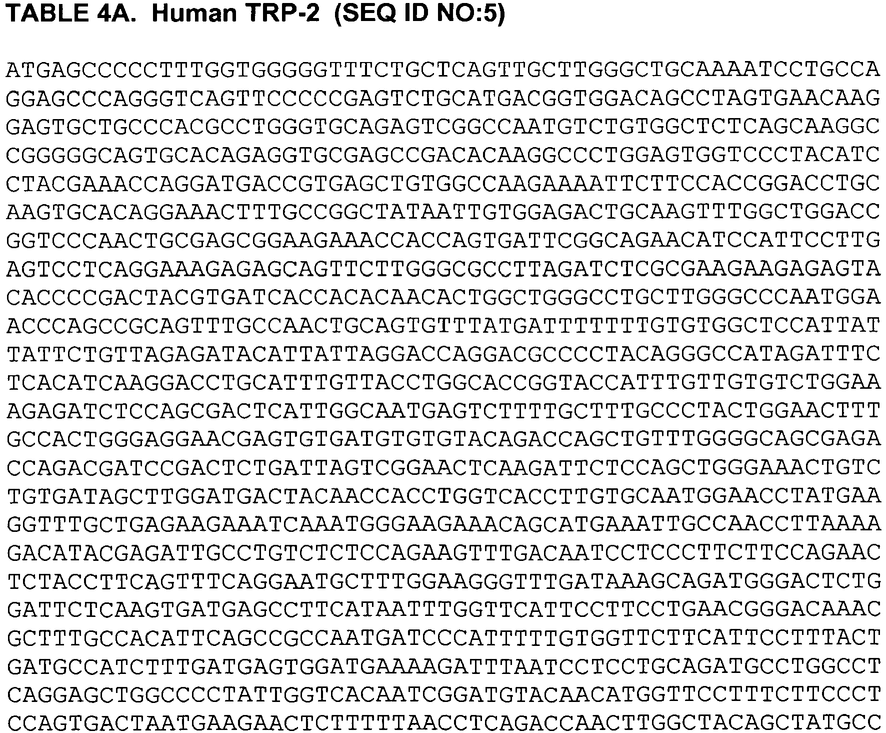

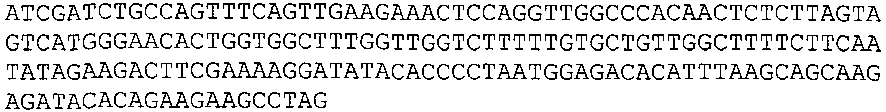

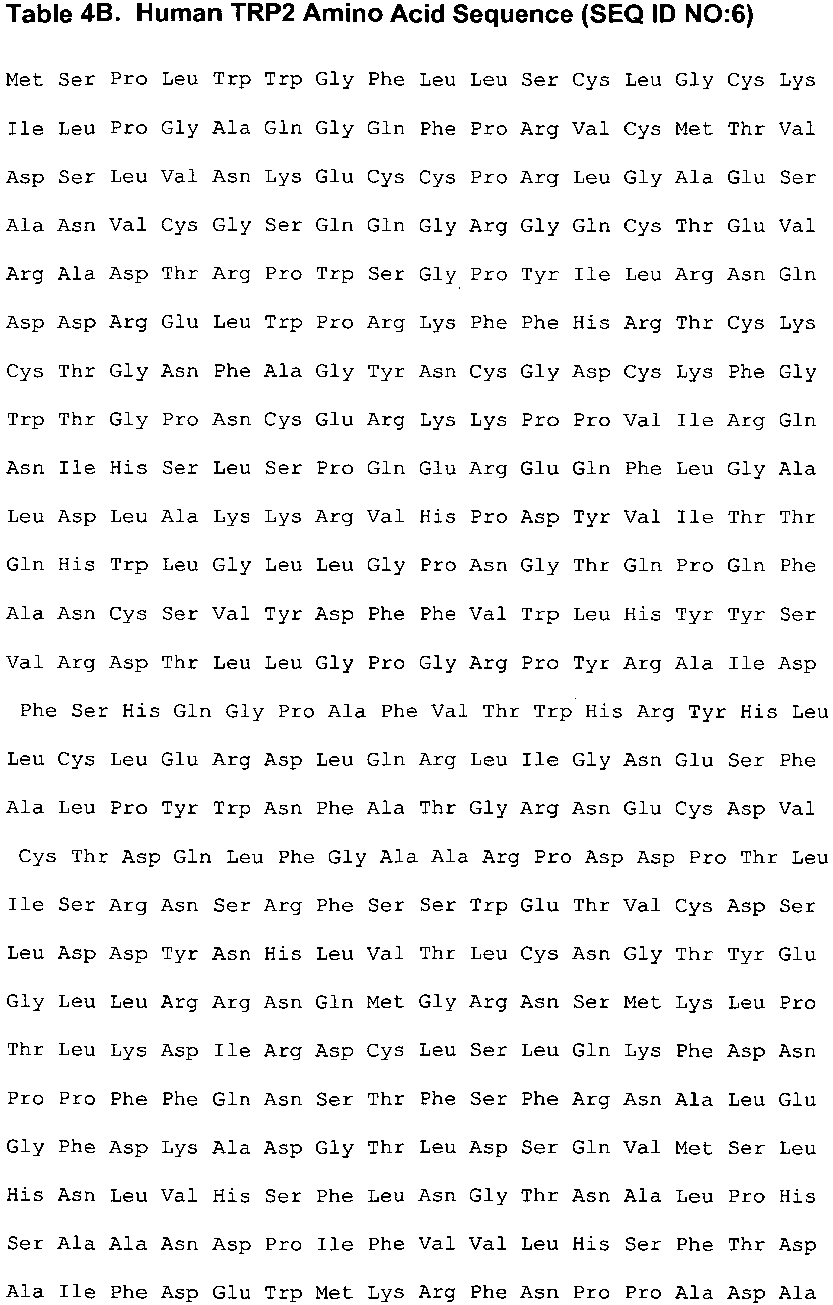

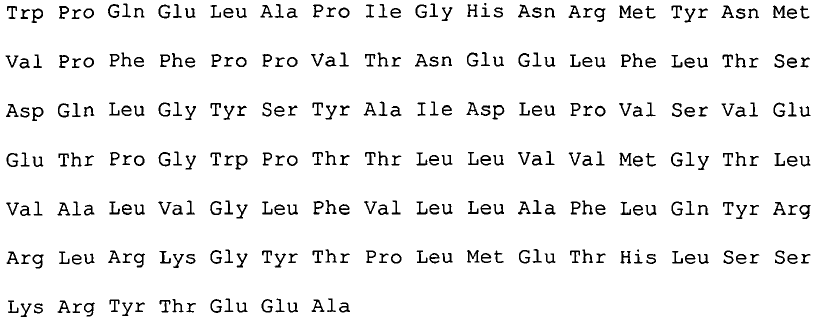

Various TRP2 sequences are available to the public via the worldwide web. Examples on the NCBI site include, but are not limited to, human (NM_01129889, DQ 902581, BC028311, L 18967, DQ894649.3, DQ891466), sheep (NM_001130024,) pig (AB207241.1), cattle (NM_00101012666, AY278108), horse (XM_001491619), macaque (M_001083014, XM_001083129, XM_001082890) and dog (XM_542639.2).

## Claims

1. A pharmaceutical composition comprising an effective amount of alphavirus replicon particles which direct expression of dopachrome tautomerase (TRP2) in a human or animal subject, for use in enhancing or inducing an immune response to melanoma cells in the subject, wherein there are no melanoma antigens other than the TRP2, and no nucleic acids which direct expression of melanoma antigens other than the TRP2, in the pharmaceutical composition.

2. The pharmaceutical composition for use of claim 1, wherein the alphavirus replicon particle is a Venezuelan Equine Encephalitis (VEE) virus replicon particle;
wherein the alphavirus is, optionally, an attenuated alphavirus;
wherein the attenuated alphavirus is, further optionally, TC-83 VEE.

3. A pharmaceutical composition comprising an effective amount of alphavirus replicon particles expressing dopachrome tautomerase (TRP2), for use in a method of reducing the risk of contracting melanoma in a human or animal subject or reducing the severity or delaying progression of melanoma in a human or animal subject with melanoma, wherein there are no melanoma antigens other than the TRP2, and no nucleic acids which direct expression of melanoma antigens other than the TRP2, in the pharmaceutical composition.

4. An immunogenic composition comprising alphavirus replicon particles which express dopachrome tautomerase (TRP2), wherein there are no melanoma antigens other than the TRP2, and no nucleic acids which direct expression of melanoma antigens other than the TRP2, in the immunogenic composition, and a pharmaceutically acceptable carrier.

5. The pharmaceutical composition for use of claim 3 or the immunogenic composition of claim 4, wherein the alphavirus replicon particle is a Venezuelan Equine Encephalitis (VEE) virus replicon particle.

6. The pharmaceutical composition for use of claim 3 or the immunogenic composition of claim 4, wherein the alphavirus replicon particle is a Venezuelan Equine Encephalitis (VEE) virus replicon particle;
wherein the VEE is an attenuated VEE.

7. The pharmaceutical composition for use of any of claims 1, 3, or claim 5 or 6 when dependent on claim 3, wherein the pharmaceutical composition is administered subcutaneously, intramuscularly, intradermally or intravenously;
wherein the pharmaceutical composition is, optionally, administered subcutaneously or intramuscularly.

8. The pharmaceutical composition for use of claim 1 or 3 or claim 7 when dependent on claim 3, wherein the TRP2 is derived in sequence from the same species as the subject.

9. The immunogenic composition of claim 4 further comprising an immunological adjuvant.

10. The immunogenic composition of claim 4 further comprising an immunological adjuvant, and further comprising a cytokine.

11. The immunogenic composition of any one of claims 4 - 6, 9 or 10, for use in a method of reducing melanoma tumor size, reducing or delaying the recurrence of a melanoma tumor or melanoma metastasis, and/or reducing or preventing metastasis of melanoma in a subject, said use comprising the step of administering a single dose of the immunogenic composition.

12. The pharmaceutical composition for use of any of claims 1 to 3 and claims 5 to 8 when dependent on claim 3, further comprising a subsequent step of administering a second dose of a pharmaceutical composition comprising TRP2 protein or comprising a nucleic acid capable of expressing TRP2 in the subject.

13. The pharmaceutical composition for use of claim 12, wherein the modality of the second dose is selected from the group consisting of protein, inactivated virus, DNA, viral-vectored antigens, alphavirus replicon particles and virus-like particles displaying or expressing TRP2.

14. The pharmaceutical composition for use of any of claims 1 to 3 and claims 5 to 8 when dependent on claim 3, further comprising at least one subsequent step of administering a pharmaceutical composition comprising Venezuelan equine encephalitis virus replicon particles which express TRP2.

15. The pharmaceutical composition for use of any of claims 1 to 3 and claims 5 to 8 when dependent on claim 3 or the immunogenic composition or the pharmaceutical composition for use of any of claims 11 to 14, wherein the pharmaceutical composition administered to a subject in a dose is from 10⁴ to 10¹⁰ virus replicon particles;
wherein the dose is, optionally, 10⁷, 5 x 10⁷, 10⁸, 5 x 10⁸, 10⁹, 5 x 10⁹, or 10¹⁰ virus replicon particles.

## Patentansprüche

1. Pharmazeutische Zusammensetzung, die eine wirksame Menge an Alphavirus-Replikon-Partikeln umfasst, die die Expression von Dopachrom-Tautomerase (TRP2) bei einem menschlichen oder tierischen Subjekt steuern, für die Verwendung bei der Stärkung oder Induzierung einer Immunantwort auf Melanomzellen bei dem Subjekt, worin keine Melanom-Antigene außer TRP2 und keine Nukleinsäuren, die die Expression von Melanom-Antigenen außer TRP2 steuern, in der pharmazeutischen Zusammensetzung vorliegen.

2. Pharmazeutische Zusammensetzung für die Verwendung nach Anspruch 1, worin das Alphavirus-Replikon-Partikel ein Replikon-Partikel des Virus von Venezuelanischer Pferdeenzephalitis (VEE) ist;
worin der Alphavirus optional ein attenuierter Alphavirus ist;
worin der attenuierte Alphavirus weiter optional TC-83 VEE ist.

3. Pharmazeutische Zusammensetzung, die eine wirksame Menge an Alphavirus-Replikon-Partikeln umfasst, die Dopachrom-Tautomerase (TRP2) exprimieren, für die Verwendung in einem Verfahren zur Verringerung des Risikos der Erkrankung an Melanom bei einem menschlichen oder tierischen Subjekt oder zur Verringerung der Schwere oder Verzögerung der Progression von Melanom bei einem menschlichen oder tierischen Subjekt mit Melanom, worin keine Melanom-Antigene außer TRP2 und keine Nukleinsäuren, die die Expression von Melanom-Antigenen außer TRP2 steuern, in der pharmazeutischen Zusammensetzung vorliegen.

4. Immunogene Zusammensetzung, die Folgendes umfasst: Alphavirus-Replikon-Partikel, die Dopachrom-Tautomerase (TRP2) exprimieren, worin keine Melanom-Antigene außer TRP2 und keine Nukleinsäuren, die die Expression von Melanom-Antigenen außer TRP2 steuern, in der immunogenen Zusammensetzung vorliegen, und einen pharmazeutisch verträglichen Träger.

5. Pharmazeutische Zusammensetzung für die Verwendung nach Anspruch 3 oder die immunogene Zusammensetzung nach Anspruch 4, worin das Alphavirus-Replikon-Partikel ein Replikon-Partikel des Virus von Venezuelanischer Pferdeenzephalitis (VEE) ist.

6. Pharmazeutische Zusammensetzung für die Verwendung nach Anspruch 3 oder die immunogene Zusammensetzung nach Anspruch 4, worin das Alphavirus-Replikon-Partikel ein Replikon-Partikel des Virus von Venezuelanischer Pferdeenzephalitis (VEE) ist;
worin die VEE eine attenuierte VEE ist.

7. Pharmazeutische Zusammensetzung für die Verwendung nach einem der Ansprüche 1, 3 oder Anspruch 5 oder 6, bei Abhängigkeit von Anspruch 3, worin die pharmazeutische Zusammensetzung subkutan, intramuskulär, intradermal oder intravenös verabreicht wird;
worin die pharmazeutische Zusammensetzung optional subkutan oder intramuskulär verabreicht wird.

8. Pharmazeutische Zusammensetzung für die Verwendung nach Anspruch 1 oder 3 oder Anspruch 7, bei Abhängigkeit von Anspruch 3, worin das TRP-2 in der Sequenz von der gleichen Spezies stammt wie das Subjekt.

9. Immunogene Zusammensetzung nach Anspruch 4, die weiter ein immunologisches Adjuvans umfasst.

10. Immunogene Zusammensetzung nach Anspruch 4, die weiter ein immunologisches Adjuvans umfasst und weiter ein Zytokin umfasst.

11. Immunogene Zusammensetzung nach einem der Ansprüche 4-6, 9 oder 10 für die Verwendung in einem Verfahren zur Verringerung der Tumorgröße eines Melanoms, Verringerung oder Verzögerung des Wiederauftretens eines Melanomtumors oder von Melanommetastase und/oder Verringerung oder Prävention von Metastase des Melanoms bei einem Subjekt, wobei die genannte Verwendung den Schritt der Verabreichung einer einzigen Dosis der immunogenen Zusammensetzung umfasst.

12. Pharmazeutische Zusammensetzung für die Verwendung nach einem der Ansprüche 1 bis 3 und der Ansprüche 5 bis 8, bei Abhängigkeit von Anspruch 3, die weiter einen nachfolgenden Schritt der Verabreichung einer zweiten Dosis einer pharmazeutischen Zusammensetzung umfasst, die TRP-2-Protein umfasst oder eine Nukleinsäure, die TRP-2 in dem Subjekt exprimieren kann, umfasst.

13. Pharmazeutische Zusammensetzung für die Verwendung nach Anspruch 12, worin die Modalität der zweiten Dosis aus der Gruppe ausgewählt ist, die aus Folgenden besteht: Protein, inaktiviertem Virus, DNA, viral-vektoriellen Antigenen, Alphavirus-Replikon-Partikeln und virusähnlichen Partikeln, die TRP-2 präsentieren oder exprimieren.

14. Pharmazeutische Zusammensetzung für die Verwendung nach einem der Ansprüche 1 bis 3 und der Ansprüche 5 bis 8, bei Abhängigkeit von Anspruch 3, die weiter mindestens einen nachfolgenden Schritt der Verabreichung einer pharmazeutischen Zusammensetzung umfasst, die Replikon-Partikel des Virus von Venezuelanischer Pferdeenzephalitis umfasst, die TRP-2 exprimieren.

15. Pharmazeutische Zusammensetzung für die Verwendung nach einem der Ansprüche 1 bis 3 und der Ansprüche 5 bis 8, bei Abhängigkeit von Anspruch 3, oder die immunogene Zusammensetzung oder die pharmazeutische Zusammensetzung für die Verwendung nach einem der Ansprüche 11 bis 14, worin die pharmazeutische Zusammensetzung, die an ein Subjekt verabreicht wird, in einer Dosis von 10⁴ bis 10¹⁰ Virus-Replikon-Partikel vorliegt;
worin die Dosis optional 10⁷, 5 x 10⁷, 10⁸, 5 x 10⁸, 10⁹, 5 x 10⁹ oder 10¹⁰ Virus-Replikon-Partikel beträgt.

## Revendications

1. Composition pharmaceutique comprenant une quantité efficace de particules d'un réplicon d'un alphavirus qui dirige l'expression de la dopachrome-tautomérase (TRP2) chez un sujet humain ou animal, pour une utilisation dans le renforcement ou l'induction d'une réponse immunitaire à des cellules mélanomateuses chez le sujet, aucun antigène du mélanome autre que la protéine TRP2 et aucun acide nucléique dirigeant l'expression d'antigènes du mélanome autre que celle de la protéine TRP2 n'étant présents dans la composition pharmaceutique.

2. Composition pharmaceutique pour l'utilisation de la revendication 1, dans laquelle la particule d'un réplicon d'un alphavirus est une particule d'un réplicon d'un virus de l'encéphalite équine du Venezuela (VEE) ;
dans laquelle l'alphavirus est en option un alphavirus atténué ;
dans laquelle l'alphavirus atténué est en outre en option la souche TC-83 du VEE.

3. Composition pharmaceutique comprenant une quantité efficace de particules d'un réplicon d'un alphavirus dirigeant l'expression de la dopachrome-tautomérase (TRP2), pour une utilisation dans une méthode de réduction du risque de développement d'un mélanome chez un sujet humain ou animal ou de réduction de la sévérité ou de retardement de la progression d'un mélanome chez un sujet humain ou animal présentant un mélanome, aucun antigène du mélanome autre que la protéine TRP2 et aucun acide nucléique dirigeant l'expression d'antigènes du mélanome autre que celle de la protéine TRP2 n'étant présents dans la composition pharmaceutique.

4. Composition immunogène comprenant des particules d'un réplicon d'un alphavirus exprimant la dopachrome-tautomérase (TRP2), aucun antigène du mélanome autre que la protéine TRP2 et aucun acide nucléique dirigeant l'expression d'antigènes du mélanome autre que celle de la protéine TRP2 n'étant présents dans la composition immunogène, et un véhicule pharmaceutiquement acceptable.

5. Composition pharmaceutique pour l'utilisation de la revendication 3 ou composition immunogène de la revendication 4, dans laquelle la particule d'un réplicon d'un alphavirus est une particule d'un réplicon d'un virus de l'encéphalite équine du Venezuela (VEE) ;

6. Composition pharmaceutique pour l'utilisation de la revendication 3 ou composition immunogène de la revendication 4, dans laquelle la particule d'un réplicon d'un alphavirus est une particule d'un réplicon d'un virus de l'encéphalite équine du Venezuela (VEE) ;
dans laquelle le VEE est un VEE atténué.

7. Composition pharmaceutique pour l'utilisation de l'une quelconque des revendications 1, 3 ou de la revendication 5 ou 6 quand dépendante de la revendication 3, dans laquelle la composition pharmaceutique est administrée par voie sous-cutanée, intramusculaire, intradermique ou intraveineuse ;
dans laquelle la composition pharmaceutique est en option administrée par voie sous-cutanée ou intramusculaire.

8. Composition pharmaceutique pour l'utilisation de la revendication 1 ou 3 ou de la revendication 7 quand dépendante de la revendication 3, dans laquelle la protéine TRP2 est dérivée en termes de séquence de la même espèce que le sujet.

9. Composition immunogène de la revendication 4, qui comprend également un adjuvant immunologique.

10. Composition immunogène de la revendication 4, qui comprend également un adjuvant immunologique et aussi une cytokine.

11. Composition immunogène de l'une quelconque des revendications 4 - 6, 9 ou 10 pour une utilisation dans une méthode de réduction de la taille d'une tumeur de type mélanome, de réduction ou de retardement de la récurrence d'une tumeur de type mélanome ou de la métastase d'un mélanome et/ou de réduction ou de prévention de la métastase d'un mélanome chez un sujet, ladite utilisation comprenant l'étape d'administration d'une dose unique de la composition immunogène.

12. Composition pharmaceutique pour l'utilisation de l'une quelconque des revendications 1 à 3 et des revendications 5 à 8 quand dépendantes de la revendication 3, qui comprend également l'étape ultérieure d'administration d'une deuxième dose d'une composition pharmaceutique comprenant la protéine TRP2 ou comprenant un acide nucléique capable d'exprimer la protéine TRP2 chez le sujet.

13. Composition pharmaceutique pour l'utilisation de la revendication 12, dans laquelle la modalité de la deuxième dose est sélectionnée dans le groupe consistant en une protéine, un virus inactivé, un ADN, des antigènes à vectorisation virale, des particules d'un réplicon d'un alphavirus et des pseudo-particules virales présentant ou exprimant la protéine TRP2.

14. Composition pharmaceutique pour l'utilisation de l'une quelconque des revendications 1 à 3 et des revendications 5 à 8 quand dépendantes de la revendication 3, qui comprend en outre au moins une étape ultérieure d'administration d'une composition pharmaceutique comprenant des particules d'un réplicon d'un virus de l'encéphalite équine du Venezuela exprimant la protéine TRP2.

15. Composition pharmaceutique pour l'utilisation de l'une quelconque des revendications 1 à 3 et des revendications 5 à 8 quand dépendantes de la revendication 3 ou la composition immunogène ou la composition pharmaceutique pour l'utilisation de l'une quelconque des revendications 11 à 14, dans laquelle la composition pharmaceutique est administrée à un sujet à une dose allant de 10⁴ à 10¹⁰ particules d'un réplicon du virus ;
dans laquelle la dose est en option de 10⁷, 5 x 10⁷, 10⁸, 5 x 10⁸, 10⁹, 5 x 10⁹ or 10¹⁰ particules d'un réplicon du virus.


### SEQUENCE LISTING

<110> AlphaVax, Inc. Wolchok, Jedd D. Avogadri, Francesca Olmsted, Robert A. Maughan, Maureen
<120> ALPHAVIRUS REPLICON PARTICLES EPXRESSING TRP2 AND METHODS
<130> 41-09 WO
<140> unassigned
   <141> 2010-04-01
<150> US 61/167,774
   <151> 2009-04-08
<160> 10
<170> PatentIn version 3.5
<210> 1
   <211> 517
   <212> PRT
   <213> Mus musculus
<400> 1
   Met Gly Leu Val Gly Trp Gly Leu Leu Leu Gly Cys Leu Gly Cys Gly
   1               5                   10                  15
   Ile Leu Leu Arg Ala Arg Ala Gln Phe Pro Arg Val Cys Met Thr Leu
               20                  25                  30
   Asp Gly Val Leu Asn Lys Glu Cys Cys Pro Pro Leu Gly Pro Glu Ala
           35                  40                  45
   Thr Asn Ile Cys Gly Phe Leu Glu Gly Arg Gly Gln Cys Ala Glu Val
       50                  55                  60
   Gln Thr Asp Thr Arg Pro Trp Ser Gly Pro Tyr Ile Leu Arg Asn Gln
   65                  70                  75                  80
   Asp Asp Arg Glu Gln Trp Pro Arg Lys Phe Phe Asn Arg Thr Cys Lys
                   85                  90                  95
   Cys Thr Gly Asn Phe Ala Gly Tyr Asn Cys Gly Gly Cys Lys Phe Gly
               100                 105                 110
   Trp Thr Gly Pro Asp Cys Asn Arg Lys Lys Pro Ala Ile Leu Arg Arg
           115                 120                 125
   Asn Ile His Ser Leu Thr Ala Gln Glu Arg Glu Gln Phe Leu Gly Ala
       130                 135                 140
   Leu Asp Leu Ala Lys Lys Ser Ile His Pro Asp Tyr Val Ile Thr Thr
   145 150 155 160
   Gln His Trp Leu Gly Leu Leu Gly Pro Asn Gly Thr Gln Pro Gln Ile
   165 170 175
   Ala Asn Cys Ser Val Tyr Asp Phe Phe Val Trp Leu His Tyr Tyr Ser
   180 185 190
   Val Arg Asp Thr Leu Leu Gly Pro Gly Arg Pro Tyr Lys Ala Ile Asp
   195 200 205
   Phe Ser His Gln Gly Pro Ala Phe Val Thr Trp His Arg Tyr His Leu
   210 215 220
   Leu Trp Leu Glu Arg Glu Leu Gln Arg Leu Thr Gly Asn Glu Ser Phe
   225 230 235 240
   Ala Leu Pro Tyr Trp Asn Phe Ala Thr Gly Lys Asn Glu Cys Asp Val
   245 250 255
   Cys Thr Asp Asp Trp Leu Gly Ala Ala Arg Gln Asp Asp Pro Thr Leu
   260 265 270
   Ile Ser Arg Asn Ser Arg Phe Ser Thr Trp Glu Ile Val Cys Asp Ser
   275 280 285
   Leu Asp Asp Tyr Asn Arg Arg Val Thr Leu Cys Asn Gly Thr Tyr Glu
   290 295 300
   Gly Leu Leu Arg Arg Asn Lys Val Gly Arg Asn Asn Glu Lys Leu Pro
   305 310 315 320
   Thr Leu Lys Asn Val Gln Asp Cys Leu Ser Leu Gln Lys Phe Asp Ser
   325 330 335
   Pro Pro Phe Phe Gln Asn Ser Thr Phe Ser Phe Arg Asn Ala Leu Glu
   340 345 350
   Gly Phe Asp Lys Ala Asp Gly Thr Leu Asp Ser Gln Val Met Asn Leu
   355 360 365
   His Asn Leu Ala His Ser Phe Leu Asn Gly Thr Asn Ala Leu Pro His
   370 375 380
   Ser Ala Ala Asn Asp Pro Val Phe Val Val Leu His Ser Phe Thr Asp
   385 390 395 400
   Ala Ile Phe Asp Glu Trp Leu Lys Arg Asn Asn Pro Ser Thr Asp Ala
   405 410 415
   Trp Pro Gln Glu Leu Ala Pro Ile Gly His Asn Arg Met Tyr Asn Met
   420 425 430
   Val Pro Phe Phe Pro Pro Val Thr Asn Glu Glu Leu Phe Leu Thr Ala
   435 440 445
   Glu Gln Leu Gly Tyr Asn Tyr Ala Val Asp Leu Ser Glu Glu Glu Ala
   450 455 460
   Pro Val Trp Ser Thr Thr Leu Ser Val Val Ile Gly Ile Leu Gly Ala
   465 470 475 480
   Phe Val Leu Leu Leu Gly Leu Leu Ala Phe Leu Gln Tyr Arg Arg Leu
   485 490 495
   Arg Lys Gly Tyr Ala Pro Leu Met Glu Thr Gly Leu Ser Ser Lys Arg
   500 505 510
   Tyr Thr Glu Glu Ala
   515
<210> 2
   <211> 1554
   <212> DNA
   <213> Mus musculus
<400> 2
   atgggacttg taggatgggg acttttgttg ggatgtttgg gatgtggaat cctgctgcgc 60
   gcccgagctc aattccccag agtgtgtatg acccttgacg gggtgctgaa caaagaatgc 120
   tgtcctcccc tcggcccaga ggcaactaat atttgcggct tcttggaagg caggggacag 180
   tgtgccgagg tccagaccga tacaaggccc tggtccgggc catacattct tcggaatcaa 240
   gatgacaggg aacagtggcc tcggaagttc ttcaaccgga cctgcaaatg cacaggaaat 300
   tttgcagggt ataattgcgg cggatgtaag ttcgggtgga ctggcccaga ttgtaataga 360
   aagaagcctg ctatcctgag gcggaacatt cacagtttga cagctcagga gagagagcag 420
   tttctgggtg ccctcgattt ggccaagaag agtattcatc ctgattatgt gataactact 480
   caacattggc tgggactgct cggtccaaac gggacacaac ctcagatcgc caactgttct 540
   gtgtacgact tcttcgtgtg gcttcactat tacagcgtcc gggacactct cctcggacct 600
   gggcgccctt ataaagcaat cgacttctcc catcagggtc cagcatttgt cacttggcac 660
   cgctaccatc tgctctggct tgagcgcgag ttgcagcgac tgaccgggaa tgagtcattt 720
   gcactgcctt actggaattt tgcaacaggc aagaatgagt gtgatgtttg cactgatgat 780
   tggctcggag ccgcaaggca ggatgaccct actctcatca gccggaatag ccggttttcc 840
   acctgggaga ttgtgtgtga tagtttggat gactacaaca ggcgcgtgac actgtgtaat 900
   gggacatacg agggactcct gcgccggaat aaggtgggac gcaacaatga aaagctgccc 960
   acgctgaaga atgtgcagga ttgcctgagc cttcagaaat tcgattcccc accattcttt 1020
   cagaactcca ccttctcttt tcgaaatgca cttgaggggt ttgacaaggc cgatgggact 1080
   ttggattctc aggttatgaa tttgcacaat ctggcgcaca gcttcctgaa cggaaccaat 1140
   gcattgccgc actccgctgc aaacgatccc gtgtttgtgg tcctccattc ctttacggac 1200
   gctatatttg atgaatggtt gaaaagaaat aatccttcaa ccgacgcgtg gccccaagag 1260
   cttgctccga ttggccataa caggatgtat aatatggtgc ccttctttcc tcccgtgact 1320
   aatgaagagc ttttcttgac cgcagagcag ctcggctata attatgccgt agaccttagt 1380
   gaggaagagg ctcccgtgtg gagcaccact ctctcagtgg tgatagggat cttgggcgcg 1440
   tttgtactgc tgctgggcct gcttgccttc ttgcagtaca ggaggctgag gaagggatat 1500
   gctcctttga tggaaaccgg tttgtctagc aaaagatata ccgaggaagc atga 1554
<210> 3
   <211> 4743
   <212> DNA
   <213> Homo sapiens
<400> 3
   aactgagttc aaggcaatta aagtcaagag ctaaggaggg agggagaggg tttagaaata     60
   ccagcataat aagtagtatg actgggtgct ctgtaaatta actcaattag acaaagcctg    120
   acttaacggg ggaagatggt gagaagcgct accctcatta aatttggttg ttagaggcgc    180
   ttctaaggaa attaagtctg ttagttgttt gaatcacata aaattgtgtg tgcacgttca    240
   tgtacacatg tgcacacatg taacctctgt gattcttgtg ggtatttttt taagaagaaa    300
   ggaatagaaa gcaaagaaaa ataaaaaata ctgaaaagaa aagactgaaa gagtagaaga    360
   taaggagaaa agtacgacag agacaaggaa agtaagagag agagagagct ctcccaatta     420
   taaagccatg agcccccttt ggtgggggtt tctgctcagt tgcttgggct gcaaaatcct     480
   gccaggagcc cagggtcagt tcccccgagt ctgcatgacg gtggacagcc tagtgaacaa    540
   ggagtgctgc ccacgcctgg gtgcagagtc ggccaatgtc tgtggctctc agcaaggccg    600
   ggggcagtgc acagaggtgc gagccgacac aaggccctgg agtggtccct acatcctacg    660
   aaaccaggat gaccgtgagc tgtggccaag aaaattcttc caccggacct gcaagtgcac    720
   aggaaacttt gccggctata attgtggaga ctgcaagttt ggctggaccg gtcccaactg    780
   cgagcggaag aaaccaccag tgattcggca gaacatccat tccttgagtc ctcaggaaag    840
   agagcagttc ttgggcgcct tagatctcgc gaagaagaga gtacaccccg actacgtgat    900
   caccacacaa cactggctgg gcctgcttgg gcccaatgga acccagccgc agtttgccaa    960
   ctgcagtgtt tatgattttt ttgtgtggct ccattattat tctgttagag atacattatt   1020
   aggaccagga cgcccctaca gggccataga tttctcacat caaggacctg catttgttac 1080
   ctggcaccgg taccatttgt tgtgtctgga aagagatctc cagcgactca ttggcaatga 1140
   gtcttttgct ttgccctact ggaactttgc cactgggagg aacgagtgtg atgtgtgtac 1200
   agaccagctg tttggggcag cgagaccaga cgatccgact ctgattagtc ggaactcaag 1260
   attctccagc tgggaaactg tctgtgatag cttggatgac tacaaccacc tggtcacctt 1320
   gtgcaatgga acctatgaag gtttgctgag aagaaatcaa atgggaagaa acagcatgaa 1380
   attgccaacc ttaaaagaca tacgagattg cctgtctctc cagaagtttg acaatcctcc 1440
   cttcttccag aactctacct tcagtttcag gaatgctttg gaagggtttg ataaagcaga 1500
   tgggactctg gattctcaag tgatgagcct tcataatttg gttcattcct tcctgaacgg 1560
   gacaaacgct ttgccacatt cagccgccaa tgatcccatt tttgtggtga tttctaatcg 1620
   tttgctttac aatgctacaa caaacatcct tgaacatgta agaaaagaga aagcgaccaa 1680
   ggaactccct tccctgcatg tgctggttct tcattccttt actgatgcca tctttgatga 1740
   gtggatgaaa agatttaatc ctcctgcaga tgcctggcct caggagctgg cccctattgg 1800
   tcacaatcgg atgtacaaca tggttccttt cttccctcca gtgactaatg aagaactctt 1860
   tttaacctca gaccaacttg gctacagcta tgccatcgat ctgccagttt cagttgaaga 1920
   aactccaggt tggcccacaa ctctcttagt agtcatggga acactggtgg ctttggttgg 1980
   tctttttgtg ctgttggctt ttcttcaata tagaagactt cgaaaaggat atacacccct 2040
   aatggagaca catttaagca gcaagagata cacagaagaa gcctagggtg ctcatgcctt 2100
   acctaagaga agaggctggc caagccacag ttctgacgct gacaataaag gaactaatcc 2160
   tcactgttcc ttcttgagtt gaagatcttt gacataggtt cttctatagt gatgatgatc 2220
   tcattcagaa gatgcttagc tgtagtttcc gctttgcttg cttgtttaac aaacccaact 2280
   aaagtgcttg aggctacctc taccttcaaa taaagataga cctgacaatt tgtgatatct 2340
   aactgagttc aaggcaatta aagtcaagag ctaaggaggg agggagaggg tttagaaata 2400
   ccagcataat aagtagtatg actgggtgct ctgtaaatta actcaattag acaaagcctg 2460
   acttaacggg ggaagatggt gagaagcgct accctcatta aatttggttg ttagaggcgc 2520
   ttctaaggaa attaagtctg ttagttgttt gaatcacata aaattgtgtg tgcacgttca 2580
   tgtacacatg tgcacacatg taacctctgt gattcttgtg ggtatttttt taagaagaaa 2640
   ggaatagaaa gcaaagaaaa ataaaaaata ctgaaaagaa aagactgaaa gagtagaaga 2700
   taaggagaaa agtacgacag agacaaggaa agtaagagag agagagagct ctcccaatta 2760
   taaagccatg agcccccttt ggtgggggtt tctgctcagt tgcttgggct gcaaaatcct 2820
   gccaggagcc cagggtcagt tcccccgagt ctgcatgacg gtggacagcc tagtgaacaa 2880
   ggagtgctgc ccacgcctgg gtgcagagtc ggccaatgtc tgtggctctc agcaaggccg 2940
   ggggcagtgc acagaggtgc gagccgacac aaggccctgg agtggtccct acatcctacg 3000
   aaaccaggat gaccgtgagc tgtggccaag aaaattcttc caccggacct gcaagtgcac 3060
   aggaaacttt gccggctata attgtggaga ctgcaagttt ggctggaccg gtcccaactg 3120
   cgagcggaag aaaccaccag tgattcggca gaacatccat tccttgagtc ctcaggaaag 3180
   agagcagttc ttgggcgcct tagatctcgc gaagaagaga gtacaccccg actacgtgat 3240
   caccacacaa cactggctgg gcctgcttgg gcccaatgga acccagccgc agtttgccaa 3300
   ctgcagtgtt tatgattttt ttgtgtggct ccattattat tctgttagag atacattatt 3360
   aggaccagga cgcccctaca gggccataga tttctcacat caaggacctg catttgttac 3420
   ctggcaccgg taccatttgt tgtgtctgga aagagatctc cagcgactca ttggcaatga 3480
   gtcttttgct ttgccctact ggaactttgc cactgggagg aacgagtgtg atgtgtgtac 3540
   agaccagctg tttggggcag cgagaccaga cgatccgact ctgattagtc ggaactcaag 3600
   attctccagc tgggaaactg tctgtgatag cttggatgac tacaaccacc tggtcacctt 3660
   gtgcaatgga acctatgaag gtttgctgag aagaaatcaa atgggaagaa acagcatgaa 3720
   attgccaacc ttaaaagaca tacgagattg cctgtctctc cagaagtttg acaatcctcc 3780
   cttcttccag aactctacct tcagtttcag gaatgctttg gaagggtttg ataaagcaga 3840
   tgggactctg gattctcaag tgatgagcct tcataatttg gttcattcct tcctgaacgg 3900
   gacaaacgct ttgccacatt cagccgccaa tgatcccatt tttgtggtga tttctaatcg 3960
   tttgctttac aatgctacaa caaacatcct tgaacatgta agaaaagaga aagcgaccaa 4020
   ggaactccct tccctgcatg tgctggttct tcattccttt actgatgcca tctttgatga 4080
   gtggatgaaa agatttaatc ctcctgcaga tgcctggcct caggagctgg cccctattgg 4140
   tcacaatcgg atgtacaaca tggttccttt cttccctcca gtgactaatg aagaactctt 4200
   tttaacctca gaccaacttg gctacagcta tgccatcgat ctgccagttt cagttgaaga 4260
   aactccaggt tggcccacaa ctctcttagt agtcatggga acactggtgg ctttggttgg 4320
   tctttttgtg ctgttggctt ttcttcaata tagaagactt cgaaaaggat atacacccct 4380
   aatggagaca catttaagca gcaagagata cacagaagaa gcctagggtg ctcatgcctt 4440
   acctaagaga agaggctggc caagccacag ttctgacgct gacaataaag gaactaatcc 4500
   tcactgttcc ttcttgagtt gaagatcttt gacataggtt cttctatagt gatgatgatc 4560
   tcattcagaa gatgcttagc tgtagtttcc gctttgcttg cttgtttaac aaacccaact 4620
   aaagtgcttg aggctacctc taccttcaaa taaagataga cctgacaatt tgtgatatct 4680
   aataataacc ccccccccaa tattgattaa gcctcctcct tttctgaaag catttaaaaa 4740
   aaa 4743
<210> 4
   <211> 3911
   <212> DNA
   <213> Homo sapiens
<400> 4
   aactgagttc aaggcaatta aagtcaagag ctaaggaggg agggagaggg tttagaaata 60
   ccagcataat aagtagtatg actgggtgct ctgtaaatta actcaattag acaaagcctg 120
   acttaacggg ggaagatggt gagaagcgct accctcatta aatttggttg ttagaggcgc 180
   ttctaaggaa attaagtctg ttagttgttt gaatcacata aaattgtgtg tgcacgttca 240
   tgtacacatg tgcacacatg taacctctgt gattcttgtg ggtatttttt taagaagaaa 300
   ggaatagaaa gcaaagaaaa ataaaaaata ctgaaaagaa aagactgaaa gagtagaaga 360
   taaggagaaa agtacgacag agacaaggaa agtaagagag agagagagct ctcccaatta 420
   taaagccatg agcccccttt ggtgggggtt tctgctcagt tgcttgggct gcaaaatcct       480
   gccaggagcc cagggtcagt tcccccgagt ctgcatgacg gtggacagcc tagtgaacaa       540
   ggagtgctgc ccacgcctgg gtgcagagtc ggccaatgtc tgtggctctc agcaaggccg 600
   ggggcagtgc acagaggtgc gagccgacac aaggccctgg agtggtccct acatcctacg 660
   aaaccaggat gaccgtgagc tgtggccaag aaaattcttc caccggacct gcaagtgcac 720
   aggaaacttt gccggctata attgtggaga ctgcaagttt ggctggaccg gtcccaactg 780
   cgagcggaag aaaccaccag tgattcggca gaacatccat tccttgagtc ctcaggaaag 840
   agagcagttc ttgggcgcct tagatctcgc gaagaagaga gtacaccccg actacgtgat 900
   caccacacaa cactggctgg gcctgcttgg gcccaatgga acccagccgc agtttgccaa 960
   ctgcagtgtt tatgattttt ttgtgtggct ccattattat tctgttagag atacattatt 1020
   aggaccagga cgcccctaca gggccataga tttctcacat caaggacctg catttgttac 1080
   ctggcaccgg taccatttgt tgtgtctgga aagagatctc cagcgactca ttggcaatga 1140
   gtcttttgct ttgccctact ggaactttgc cactgggagg aacgagtgtg atgtgtgtac 1200
   agaccagctg tttggggcag cgagaccaga cgatccgact ctgattagtc ggaactcaag 1260
   attctccagc tgggaaactg tctgtgatag cttggatgac tacaaccacc tggtcacctt 1320
   gtgcaatgga acctatgaag gtttgctgag aagaaatcaa atgggaagaa acagcatgaa 1380
   attgccaacc ttaaaagaca tacgagattg cctgtctctc cagaagtttg acaatcctcc 1440
   cttcttccag aactctacct tcagtttcag gaatgctttg gaagggtttg ataaagcaga 1500
   tgggactcaa ctgagttcaa ggcaattaaa gtcaagagct aaggagggag ggagagggtt 1560
   tagaaatacc agcataataa gtagtatgac tgggtgctct gtaaattaac tcaattagac 1620
   aaagcctgac ttaacggggg aagatggtga gaagcgctac cctcattaaa tttggttgtt 1680
   agaggcgctt ctaaggaaat taagtctgtt agttgtttga atcacataaa attgtgtgtg 1740
   cacgttcatg tacacatgtg cacacatgta acctctgtga ttcttgtggg tattttttta 1800
   agaagaaagg aatagaaagc aaagaaaaat aaaaaatact gaaaagaaaa gactgaaaga 1860
   gtagaagata aggagaaaag tacgacagag acaaggaaag taagagagag agagagctct 1920
   cccaattata aagccatgag ccccctttgg tgggggtttc tgctcagttg cttgggctgc 1980
   aaaatcctgc caggagccca gggtcagttc ccccgagtct gcatgacggt ggacagccta 2040
   gtgaacaagg agtgctgccc acgcctgggt gcagagtcgg ccaatgtctg tggctctcag 2100
   caaggccggg ggcagtgcac agaggtgcga gccgacacaa ggccctggag tggtccctac 2160
   atcctacgaa accaggatga ccgtgagctg tggccaagaa aattcttcca ccggacctgc 2220
   aagtgcacag gaaactttgc cggctataat tgtggagact gcaagtttgg ctggaccggt 2280
   cccaactgcg agcggaagaa accaccagtg attcggcaga acatccattc cttgagtcct 2340
   caggaaagag agcagttctt gggcgcctta gatctcgcga agaagagagt acaccccgac 2400
   tacgtgatca ccacacaaca ctggctgggc ctgcttgggc ccaatggaac ccagccgcag 2460
   tttgccaact gcagtgttta tgattttttt gtgtggctcc attattattc tgttagagat 2520
   acattattag gaccaggacg cccctacagg gccatagatt tctcacatca aggacctgca 2580
   tttgttacct ggcaccggta ccatttgttg tgtctggaaa gagatctcca gcgactcatt 2640
   ggcaatgagt cttttgcttt gccctactgg aactttgcca ctgggaggaa cgagtgtgat 2700
   gtgtgtacag accagctgtt tggggcagcg agaccagacg atccgactct gattagtcgg 2760
   aactcaagat tctccagctg ggaaactgtc tgtgatagct tggatgacta caaccacctg 2820
   gtcaccttgt gcaatggaac ctatgaaggt ttgctgagaa gaaatcaaat gggaagaaac 2880
   agcatgaaat tgccaacctt aaaagacata cgagattgcc tgtctctcca gaagtttgac 2940
   aatcctccct tcttccagaa ctctaccttc agtttcagga atgctttgga agggtttgat 3000
   aaagcagatg ggactctgga ttctcaagtg atgagccttc ataatttggt tcattccttc 3060
   ctgaacggga caaacgcttt gccacattca gccgccaatg atcccatttt tgtggtgatt 3120
   tctaatcgtt tgctttacaa tgctacaaca aacatccttg aacatgtaag aaaagagaaa 3180
   gcgaccaagg aactcccttc cctgcatgtg ctggttcttc attcctttac tgatgccatc 3240
   tttgatgagt ggatgaaaag atttaatcct cctgcagatg cctggcctca ggagctggcc 3300
   cctattggtc acaatcggat gtacaacatg gttcctttct tccctccagt gactaatgaa 3360
   gaactctttt taacctcaga ccaacttggc tacagctatg ccatcgatct gccagtttca 3420
   gttgaagaaa ctccaggttg gcccacaact ctcttagtag tcatgggaac actggtggct 3480
   ttggttggtc tttttgtgct gttggctttt cttcaatata gaagacttcg aaaaggatat 3540
   acacccctaa tggagacaca tttaagcagc aagagataca cagaagaagc ctagggtgct 3600
   catgccttac ctaagagaag aggctggcca agccacagtt ctgacgctga caataaagga 3660
   actaatcctc actgttcctt cttgagttga agatctttga cataggttct tctatagtga 3720
   tgatgatctc attcagaaga tgcttagctg tagtttccgc tttgcttgct tgtttaacaa 3780
   acccaactaa agtgcttgag gctacctcta ccttcaaata aagatagacc tgacaatttg 3840
   tgatatctaa taataacccc ccccccaata ttgattaagc ctcctccttt tctgaaagca 3900
   tttaaaaaaa a 3911
<210> 5
   <211> 1560
   <212> DNA
   <213> Homo sapiens
<400> 5
   atgagccccc tttggtgggg gtttctgctc agttgcttgg gctgcaaaat cctgccagga 60
   gcccagggtc agttcccccg agtctgcatg acggtggaca gcctagtgaa caaggagtgc 120
   tgcccacgcc tgggtgcaga gtcggccaat gtctgtggct ctcagcaagg ccgggggcag 180
   tgcacagagg tgcgagccga cacaaggccc tggagtggtc cctacatcct acgaaaccag 240
   gatgaccgtg agctgtggcc aagaaaattc ttccaccgga cctgcaagtg cacaggaaac 300
   tttgccggct ataattgtgg agactgcaag tttggctgga ccggtcccaa ctgcgagcgg 360
   aagaaaccac cagtgattcg gcagaacatc cattccttga gtcctcagga aagagagcag 420
   ttcttgggcg ccttagatct cgcgaagaag agagtacacc ccgactacgt gatcaccaca 480
   caacactggc tgggcctgct tgggcccaat ggaacccagc cgcagtttgc caactgcagt 540
   gtttatgatt tttttgtgtg gctccattat tattctgtta gagatacatt attaggacca 600
   ggacgcccct acagggccat agatttctca catcaaggac ctgcatttgt tacctggcac 660
   cggtaccatt tgttgtgtct ggaaagagat ctccagcgac tcattggcaa tgagtctttt 720
   gctttgccct actggaactt tgccactggg aggaacgagt gtgatgtgtg tacagaccag 780
   ctgtttgggg cagcgagacc agacgatccg actctgatta gtcggaactc aagattctcc 840
   agctgggaaa ctgtctgtga tagcttggat gactacaacc acctggtcac cttgtgcaat 900
   ggaacctatg aaggtttgct gagaagaaat caaatgggaa gaaacagcat gaaattgcca 960
   accttaaaag acatacgaga ttgcctgtct ctccagaagt ttgacaatcc tcccttcttc 1020
   cagaactcta ccttcagttt caggaatgct ttggaagggt ttgataaagc agatgggact 1080
   ctggattctc aagtgatgag ccttcataat ttggttcatt ccttcctgaa cgggacaaac 1140
   gctttgccac attcagccgc caatgatccc atttttgtgg ttcttcattc ctttactgat 1200
   gccatctttg atgagtggat gaaaagattt aatcctcctg cagatgcctg gcctcaggag 1260
   ctggccccta ttggtcacaa tcggatgtac aacatggttc ctttcttccc tccagtgact 1320
   aatgaagaac tctttttaac ctcagaccaa cttggctaca gctatgccat cgatctgcca 1380
   gtttcagttg aagaaactcc aggttggccc acaactctct tagtagtcat gggaacactg 1440
   gtggctttgg ttggtctttt tgtgctgttg gcttttcttc aatatagaag acttcgaaaa 1500
   ggatatacac ccctaatgga gacacattta agcagcaaga gatacacaga agaagcctag 1560
<210> 6
   <211> 519
   <212> PRT
   <213> Homo sapiens
<400> 6
   Met Ser Pro Leu Trp Trp Gly Phe Leu Leu Ser Cys Leu Gly Cys Lys
   1               5                   10                  15
   Ile Leu Pro Gly Ala Gln Gly Gln Phe Pro Arg Val Cys Met Thr Val
               20                  25                  30
   Asp Ser Leu Val Asn Lys Glu Cys Cys Pro Arg Leu Gly Ala Glu Ser
           35                  40                  45
   Ala Asn Val Cys Gly Ser Gln Gln Gly Arg Gly Gln Cys Thr Glu Val
       50                  55                  60
   Arg Ala Asp Thr Arg Pro Trp Ser Gly Pro Tyr Ile Leu Arg Asn Gln
   65                  70                  75                  80
   Asp Asp Arg Glu Leu Trp Pro Arg Lys Phe Phe His Arg Thr Cys Lys
                   85                  90                  95
   Cys Thr Gly Asn Phe Ala Gly Tyr Asn Cys Gly Asp Cys Lys Phe Gly
               100                 105                 110
   Trp Thr Gly Pro Asn Cys Glu Arg Lys Lys Pro Pro Val Ile Arg Gln
           115                 120                 125
   Asn Ile His Ser Leu Ser Pro Gln Glu Arg Glu Gln Phe Leu Gly Ala
       130                 135                 140
   Leu Asp Leu Ala Lys Lys Arg Val His Pro Asp Tyr Val Ile Thr Thr
   145                 150                 155                 160
   Gln His Trp Leu Gly Leu Leu Gly Pro Asn Gly Thr Gln Pro Gln Phe
                   165                 170                 175
   Ala Asn Cys Ser Val Tyr Asp Phe Phe Val Trp Leu His Tyr Tyr Ser
               180                 185                 190
   Val Arg Asp Thr Leu Leu Gly Pro Gly Arg Pro Tyr Arg Ala Ile Asp
           195                 200                 205
   Phe Ser His Gln Gly Pro Ala Phe Val Thr Trp His Arg Tyr His Leu
       210                 215                 220
   Leu Cys Leu Glu Arg Asp Leu Gln Arg Leu Ile Gly Asn Glu Ser Phe
   225                 230                 235                 240
   Ala Leu Pro Tyr Trp Asn Phe Ala Thr Gly Arg Asn Glu Cys Asp Val
   245 250 255
   Cys Thr Asp Gln Leu Phe Gly Ala Ala Arg Pro Asp Asp Pro Thr Leu
   260 265 270
   Ile Ser Arg Asn Ser Arg Phe Ser Ser Trp Glu Thr Val Cys Asp Ser
   275 280 285
   Leu Asp Asp Tyr Asn His Leu Val Thr Leu Cys Asn Gly Thr Tyr Glu
   290 295 300
   Gly Leu Leu Arg Arg Asn Gln Met Gly Arg Asn Ser Met Lys Leu Pro
   305 310 315 320
   Thr Leu Lys Asp Ile Arg Asp Cys Leu Ser Leu Gln Lys Phe Asp Asn
   325 330 335
   Pro Pro Phe Phe Gln Asn Ser Thr Phe Ser Phe Arg Asn Ala Leu Glu
   340 345 350
   Gly Phe Asp Lys Ala Asp Gly Thr Leu Asp Ser Gln Val Met Ser Leu
   355 360 365
   His Asn Leu Val His Ser Phe Leu Asn Gly Thr Asn Ala Leu Pro His
   370 375 380
   Ser Ala Ala Asn Asp Pro Ile Phe Val Val Leu His Ser Phe Thr Asp
   385 390 395 400
   Ala Ile Phe Asp Glu Trp Met Lys Arg Phe Asn Pro Pro Ala Asp Ala
   405 410 415
   Trp Pro Gln Glu Leu Ala Pro Ile Gly His Asn Arg Met Tyr Asn Met
   420 425 430
   Val Pro Phe Phe Pro Pro Val Thr Asn Glu Glu Leu Phe Leu Thr Ser
   435 440 445
   Asp Gln Leu Gly Tyr Ser Tyr Ala Ile Asp Leu Pro Val Ser Val Glu
   450 455 460
   Glu Thr Pro Gly Trp Pro Thr Thr Leu Leu Val Val Met Gly Thr Leu
   465 470 475 480
   Val Ala Leu Val Gly Leu Phe Val Leu Leu Ala Phe Leu Gln Tyr Arg
   485 490 495
   Arg Leu Arg Lys Gly Tyr Thr Pro Leu Met Glu Thr His Leu Ser Ser
               500                 505                 510
   Lys Arg Tyr Thr Glu Glu Ala
           515
<210> 7
   <211> 38
   <212> DNA
   <213> Artificial Sequence
<220>
   <223> Synthetic construct: human TRP-2 EcoR5 forward primer
<400> 7
   gagccccctt tggtgggggt ttctgctcag ttgcttgg 38
<210> 8
   <211> 44
   <212> DNA
   <213> Artificial Sequence
<220>
   <223> Synthetic construct: human TRP-2 Xba/PacI reverse primer
<400> 8
   ggatggctct agattaatta attactaggc ttcttctgtg tatc 44
<210> 9
   <211> 38
   <212> DNA
   <213> Artificial Sequence
<220>
   <223> Synthetic construct: primer for human TRP2
<400> 9
   gagccccctt tggtgggggt ttctgctcag ttgcttgg 38
<210> 10
   <211> 44
   <212> DNA
   <213> Artificial Sequence
<220>
   <223> Synthetic construct: reverse primer for human TRP2
<400> 10
   ggatggctct agattaatta attactaggc ttcttctgtg tatc 44